# FASCICULE DE BREVET EUROPEEN

(11) **EP 0 873 993 B1**
(45) Date de publication et mention de la délivrance du brevet: **20.02.2002**
(21) Numéro de dépôt: 98400987.8
(22) Date de dépôt: 23.04.1998
(51) Int. Cl.: C07D 319/18, C07D 327/06, C07D 335/06, C07D 311/64, A61K 31/335, A61K 31/39

(54) **Nouveaux composés hétérocycliques, leur procédé de préparation et les compositions pharmaceutiques qui les contiennent**
Heterozyclische Verbindungen, Verfahren zu deren Herstellung und diese enthaltende pharmazeutische Zusammenstellungen
Heterocyclic compounds, process for their preparation and pharmaceutical compositions containing them

(30) Priorité: 25.04.1997 FR 9705110
(43) Date de publication de la demande: 28.10.1998
(73) Titulaire: LES LABORATOIRES SERVIER, 92200 Neuilly sur Seine (FR)
(72) Inventeur: Guillaumet, Gérald, 45650 Saint Jean Le Blanc (FR); Charton, Isabelle, 91330 Yerres (FR); Mamai, Ahmed, 45100 Orleans (FR); Renard, Pierre, 78000 Versailles (FR); Pfeiffer, Bruno, 95320 Saint Leu La Foret (FR); Delagrange, Philippe, 92130 Issy les Moulineaux (FR); Guardiola, Béatrice, 92210 Saint Cloud (FR)

(56) Documents cités:
- EP-A- 0 315 009
- EP-A- 0 669 331
- CHEMICAL ABSTRACTS, vol. 69, no. 12, 16 septembre 1968 Columbus, Ohio, US; abstract no. 47977p, ZURAUSKIENE,E.: "LUMINESCENCE SPECTRA OF OXYGEN DERIV. OF 1,4-BENZODIOXAN." page 4492; colonne 1; XP002072887 & LIET.FIZ. RINKINYS,LIET.TSR MOKSLU AKAD., vol. 7, no. 1, 1967, pages 213-220, RUSS

## Description

L'invention concerne de nouveaux dérivés hétérocycliques, leur procédé de préparation et les compositions pharmaceutiques qui les contiennent. Ces nouveaux composés s'avèrent être de puissants ligands des récepteurs mélatoninergiques.

On connaît dans l'art antérieur (WO 9603378) des carboxamides 2.3-dihydrobenzodioxiniques à chaîne ramifiée inhibiteurs d'Acyl CoA transférase utiles dans le traitement de l'arthériosclérose.

On connaît également de nombreux carbamates 2,3-dihydro-benzodioxiniques (EP 38945, FR 1494650) ou benzodithiiniques (US 3636047) utiles en tant qu'herbicides. insecticides ou acaricides.

Des amides 2,3-dihydro-benzodioxiniques (EP 669 331) sont par ailleurs décrits dans la littérature pour le traitement de la schizophrénie.

Des composés benzodioxiniques, chromanes ou thiochromanes directement benzo substitués par des amides ou des carboxamides sont décrits en tant qu'intermédiaires de synthèse (Journal of Heterocyclic Chemistry, 1973, 10(4), pp. 623-9 ; EP 79683) ou utiles en tant qu'agents anxiolytiques et antidépresseurs (FR 2360305), en tant qu'agents neuroleptiques (DE 3702005) ou antagonistes dopaminergiques (WO 8403281).

De nombreuses études ont mis en évidence ces dix dernières années, le rôle capital de la mélatonine (5-méthoxy N-acétyl tryptamine) dans le contrôle du rythme circadien et des fonctions endocrines. De surcroît, les récepteurs de la mélatonine ont été caractérisés et localisés.

Outre leur action bénéfique sur les troubles du rythme circadien (J. Neurosurg. 1985, 63, pp 321-341) et du sommeil (Psychopharmacology, 1990, 100, pp 222-226), les ligands du système mélatoninergique possèdent d'intéressantes propriétés pharmacologiques sur le système nerveux central, notamment anxiolytiques et antipsychotiques (Neuropharmacology of Pineal Secretions, 1990, 8 (3-4), pp 264-272) et analgésiques (Pharmacopsychiat., 1987, 20, pp 222-223) ainsi que pour le traitement des maladies de Parkinson (J. Neurosurg. 1985, 63, pp 321-341) et d'Alzheimer (Brain Research. 1990, 528, pp 170-174). De même, ces composés ont montré une activité sur certains cancers (Melatonin - Clinical Perspectives, Oxford University Press, 1988, pp 164-165), sur l'ovulation (Science 1987, 227, pp 714-720), sur le diabète (Clinical Endocrinology. 1986, 24, pp 359-364), et sur le traitement de l'obésité (International Journal of Eating Disorders, 1996, 20 (4), pp 443-446).

Des composés permettant d'agir sur le système mélatoninergique sont donc pour le clinicien d'excellents médicaments pour le traitement des pathologies liées au système mélatoninergique et notamment celles mentionnées précédemment.

L'invention concerne plus spécifiquement les composés de formule (I): 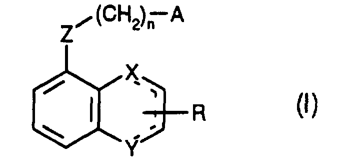 dans laquelle :
◆ X et Y, identiques ou différents, représentent un atome de soufre, un atome d'oxygène, un groupement CHq (où q vaut 0, 1 ou 2), SO ou SO₂, étant entendu que X et Y ne peuvent représenter simultanément un groupement CHq (où q vaut 0, 1 ou 2),
◆ Z représente un atome d'oxygène ou un groupement CH₂,
◆ n est égal à 0, 1, 2, 3 ou 4,
◆ A représente
   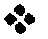 un groupement 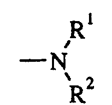 dans lequel
      R¹ représente un atome d'hydrogène ou un groupement alkyle (C₁-C₆) linéaire ou ramifié,
      et R² représente un groupement 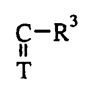 dans lequel T représente un atome de soufre ou un atome d'oxygène et R³ représente
         - un groupement R'³ représentant un atome d'hydrogène, un groupement alkyle (C₁-C₆) linéaire ou ramifié éventuellement substitué, un groupement alcényle (C₂-C₆) linéaire ou ramifié éventuellement substitué, un groupement alcynyle (C₂-C₆) linéaire ou ramifié éventuellement substitué, un groupement cycloalkyle (C₃-C₈), cycloalkyle (C₃-C₈) substitué, cycloalkylalkyle, cycloalkylalkyle substitué, aryle, arylalkyle,
         - ou un groupement 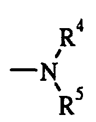
         dans lequel R⁴ représente un atome d'hydrogène ou un groupement alkyle (C₁-C₆) linéaire ou ramifié et R⁵ représente un atome d'hydrogène, un groupement R'⁵ représentant un groupement alkyle (C₁-C₆) linéaire ou ramifié éventuellement substitué, un groupement alcényle (C₂-C₆) linéaire ou ramifié éventuellement substitué, un groupement alcynyle (C₂-C₆) linéaire ou ramifié éventuellement substitué, un groupement cycloalkyle (C₃-C₈). cycloalkyle (C₃-C₈) substitué, cycloalkylalkyle, cycloalkylalkyle substitué, aryle, ou arylalkyle,
   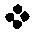 ou un groupement 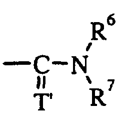 dans lequel
      T' représente un atome de soufre ou un atome d'oxygène
      R⁶ représente un atome d'hydrogène ou un groupement alkyle (C₁-C₆) linéaire ou ramifié
      et R⁷ représente un atome d'hydrogène, un groupement alkyle (C₁-C₆) linéaire ou ramifié éventuellement substitué, un groupement alcényle (C₂-C₆) linéaire ou ramifié éventuellement substitué, un groupement alcynyle (C₂-C₆) linéaire ou ramifié éventuellement substitué, un groupement cycloalkyle (C₃-C₈), cycloalkyle (C₃-C₈) substitué, cycloalkylalkyle, cycloalkylalkyle substitué, aryle, ou arylalkyle,
◆ R représente un atome d'hydrogène, un groupement alkyle (C₁-C₆) linéaire ou ramifié éventuellement substitué, un groupement aryle ou arylalkyle,
◆ la représentation 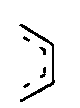 signifie que ces liaisons peuvent être simples ou doubles, étant entendu que deux liaisons adjacentes ne peuvent être doubles simultanément et que la valence des atomes est respectée,
étant entendu que :
- le terme "aryle" désigne un groupement phényle ou naphtyle éventuellement substitué par un ou plusieurs atomes d'halogène, groupements OH, alkyle (C₁-C₆) linéaire ou ramifié, alkoxy (C₁-C₆) linéaire ou ramifié, cyano, nitro, amino ou trihalogénoalkyle,
- le terme "arylalkyle" désigne un groupement alkyle (C₁-C₆) linéaire ou ramifié substitué par un groupement aryle tel que défini ci-dessus,
- le terme "cycloalkylalkyle" désigne un alkyle (C₁-C₆) linéaire ou ramifié substitué par un ou plusieurs groupements cycloalkyle (C₃-C₈),
- le terme substitué affecté aux termes "alkyle", "alcényle" et "alcynyle" signifie que ce groupement est substitué par un ou plusieurs atomes d'halogène, groupements OH, ou alkoxy,
- le terme substitué affecté aux termes "cycloalkyle" et "cycloalkylalkyle" signifie que la partie cyclique est substituée par un ou plusieurs atomes d'halogène, groupements alkyle (C₁-C₆) linéaire ou ramifié, alkoxy (C₁-C₆) linéaire ou ramifié, phényle, hydroxy ou oxo,
étant entendu que
. lorsque X et Z représentent simultanément un groupement CH₂, R représente un atome d'hydrogène et A représente un groupement NR¹R², alors Y ne peut représenter un atome d'oxygène.
. lorsque Z représente un atome d'oxygène, alors n est différent de zéro,
. lorsque Z représente un atome d'oxygène et n vaut un, alors A ne peut représenter un groupement CONEt₂,
. lorsque Z représente un groupement CH₂, n vaut 1 et A représente un groupement -NR¹CSNR⁴R⁵, alors R⁵ ne peut représenter un groupement aryle,
. lorsque X et Y représentent simultanément un atome d'oxygène, que les liaisons en pointillés sont saturées, R représente un atome d'hydrogène ou un groupement CH₂OH, alors A est différent d'un groupement NR¹R^{2a} dans lequel R¹ est tel que défini précédemment et R^{2a} représente un groupement benzoyle éventuellemen substitué,
leurs énantiomères et diastéréoisomères, ainsi que leurs sels d'addition à un acide ou une base pharmaceutiquement acceptable.

Parmi les acides pharmaceutiquement acceptables, on peut citer à titre non limitatif les acides chlorhydrique, bromhydrique, sulfurique, phosphonique, acétique, trifluoroacétique, lactique, pyruvique, malonique, succinique, glutarique, fumarique, tartrique, maléïque, citrique, ascorbique, méthane sulfonique, camphorique, etc...

Parmi les bases pharmaceutiquement acceptables, on peut citer à titre non limitatif l'hydroxyde de sodium, l'hydroxyde de potassium, la triéthylamine, la tertbutylamine, etc...

Les composés préférés de l'invention sont ceux pour lesquels :
- X et Y représentent simultanément un atome d'oxygène,
- X représente un atome de soufre et Y représente un atome d'oxygène,
- X représente un atome de soufre ou d'oxygène et Y représente un groupement CH_{q}(où q vaut 0, 1 ou 2),
- Z représente un atome d'oxygène,
- Z représente un groupement CH₂,
- R représente un atome d'hydrogène.

Les substituants A préférés de l'invention sont :
- le groupement -NR¹COR³ dans lequel R¹ et R³ sont tels que définis précédemment,
- et le groupement -CONR⁶R⁷ dans lequel R⁶ et R⁷ sont tels que définis précédemment.

Plus particulièrement, l'invention concerne les dérivés benzodioxiniques, dihydrobenzodioxiniques. benzoxathiiniques, dihydrobenzoxathiiniques, benzopyraniques, dihydrobenzopyraniques, benzothiopyraniques, dihydrobenzothiopyraniques.

Encore plus spécifiquement, l'invention concerne les composés de formule (I) qui sont :
- le N-méthyl-[4-(2,3-dihydro-1,4-benzoxathiin-5-yl)]butanamide,
- le N-éthyl-[4-(2,3-dihydro-1,4-benzoxathlin-5-yl)] butanamide,
- le N-[3-(1,4-benzodioxin-5-yl)propyl] butanamide,
- le N-méthyl-[4-(2,3-dihydro-1,4-benzodioxin-5-yl)] butanamide,

Les énantiomères, diastéréoisomères ainsi que les sels d'addition à un acide ou à une base pharmaceutiquement acceptable des composés préférés de l'invention font partie intégrante de l'invention.

L'invention s'étend également au procédé de préparation du composé de formule (I) caractérisé en ce que l'on utilise comme produit de départ un composé de formule (II) : 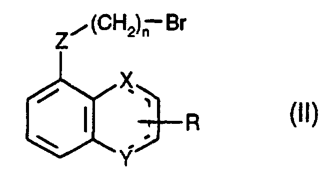 dans laquelle X, Y, Z, R et n sont tels que définis précédemment.
◆ que l'on soumet successivement à l'action d'un phtalimidure puis à une hydrazinolyse pour conduire au composé de formule (III): 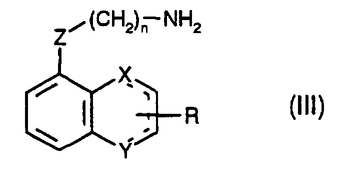 dans laquelle X, Y, Z, n et R sont tels que définis précédemment,
   composé (III) pouvant être par ailleurs obtenu à partir du composé (II) par action d'un sel de cyanure ou un azoture suivi d'une réduction,
   sur lequel on condense :
   - un chlorure d'acyle de formule (IV) 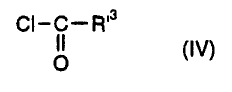 dans laquelle R'³ est tel que défini précédemment, ou l'anhydride d'acide (mixte ou symétrique) correspondant.
      pour obtenir le composé de formule (I/a), cas particulier des composés de formule (I): 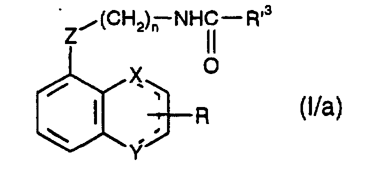 dans laquelle X, Y, Z, n, R et R'³ sont tels que définis précédemment,
      qui peut être soumis à un agent de thionation comme le réactif de Lawesson pour obtenir le composé de formule (I/b), cas particuliers des composés de formule (I) : 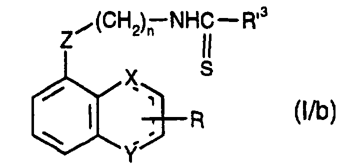 dans laquelle X. Y, Z. n, R et R'³ sont tels que définis précédemment,
   - ou un composé de formule (V)

      T=C=N-R⁵ (V)

      dans laquelle T et R⁵ sont définis comme précédemment,
      afin d'obtenir le composé de formule (I/c), cas particulier des composés de formule (I): 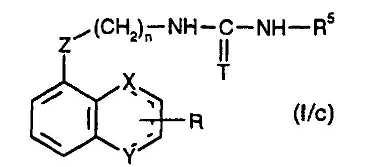 dans laquelle X, Y, Z, n, R, T et R⁵ sont définis comme précédemment,
      l'ensemble des composés (I/a), (I/b) et (I/c) formant le composé de formule (I/d), cas particulier des composés de formule (I) : 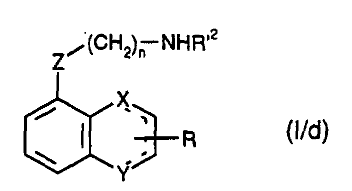 dans laquelle X, Y. Z, n et R sont tels que définis précédemment et R'² représente un groupement 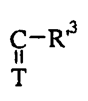 où T et R'³ sont définis comme précédemment
      ou un groupement 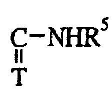 où T et R⁵ sont définis comme précédemment, composé (I/d) que l'on peut alkyler selon une technique classique d'alkylation grâce à un composé de formule (VI):

      Alk-W (VI)

      dans laquelle Alk représente un groupement alkyle (C₁-C₆) linéaire ou ramifié et W représente un groupe partant comme un atome d'halogène ou un groupement tosyle,
      ou grâce à un dialkylsulfate,
      pour conduire au composé de formule (I/e), cas particulier des composés de formule (I): 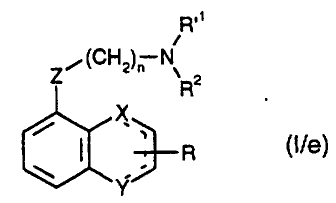 dans laquelle X. Y, Z, n, R et R² sont tels que définis précédemment, et R'¹ représente un groupement alkyle (C₁-C₆) linéaire ou ramifié,
◆ ou que l'on soumet successivement à l'action d'un sel d'acide carboxylique puis à une saponification pour conduire au composé de formule (VII): 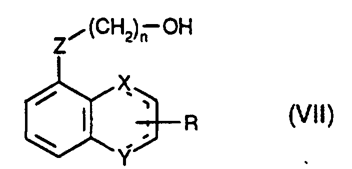 dans laquelle X, Y. Z, n et R sont tels que définis précédemment.
   qui est :
   - soit oxydé en aldéhyde correspondant pour obtenir le composé de formule (VIII) : 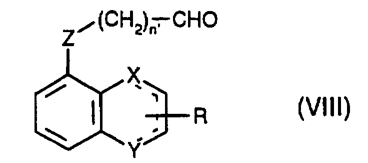 dans laquelle X, Y, Z, et R sont définis comme précédemment, et n' vaut 0, 1, 2 ou 3,
      qui est oxydé en acide correspondant puis soumis à l'action d'une amine H₂NR⁷ où R⁷ est tel que défini précédemment,
      pour conduire au composé de formule (I/f), cas particulier des composés de formule (I) : 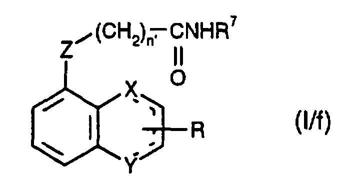 dans laquelle X, Y, Z, n', R et R⁷ sont tels que définis précédemment,
      qui peut être soumis à un agent de thionation comme le réactif de Lawesson pour obtenir le composé (I/g), cas particulier des composés de formule (I) : 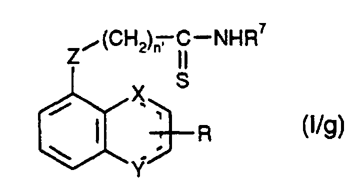 dans laquelle X, Y. Z, n', R et R⁷ sont tels que définis précédemment,
      l'ensemble des composés (I/f) et (I/g) formant le composé de formule (I/h) : 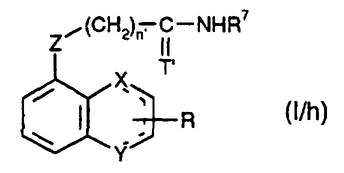 dans laquelle X, Y, Z, T', n', R et R⁷ sont tels que définis précédemment,
      composé (I/h) qui peut être alkylé selon une technique classique d'alkylation pour conduire au composé de formule (I/i), cas particulier des composés de formule (I) : 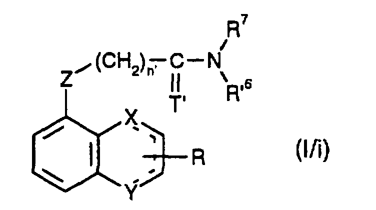 dans laquelle X. Y. Z, T', n', R et R⁷ sont tels que définis précédemment, et R'⁶ représente un groupement alkyle (C₁-C₆) linéaire ou ramifié,
   - soit transformé en groupe partant comme un tosylate puis substitué
      . par un cyanure pour conduire au composé de formule (IX) : 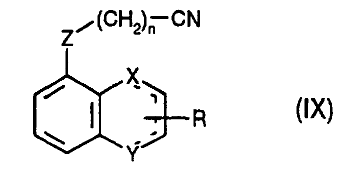 dans laquelle X, Y, Z, R et n sont tels que définis précédemment,
         qui est successivement soumis à une hydrolyse et à la condensation d'une amine H₂NR⁷ pour conduire au composé de formule (I/j) cas particulier des composés de formule (I) : 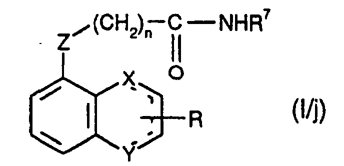 dans laquelle X, Y, Z, n, R et R⁷ sont tels que définis précédemment.
         qui peut être soumis à un agent de thionation comme le réactif de Lawesson pour obtenir le composé (I/k), cas particulier des composés de formule (I) : 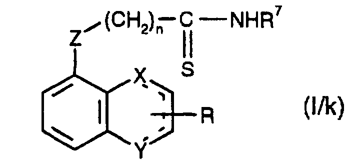 dans laquelle X, Y, Z, n, R et R⁷ sont tels que définis précédemment,
         l'ensemble des composés (I/j) et (I/k) formant le composé de formule (I/l), cas particulier des composés de formule (I) : 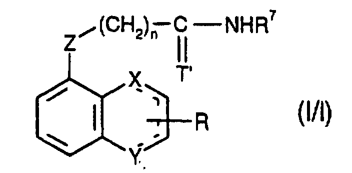 dans laquelle X. Y, Z, n, T', R et R⁷ sont tels que définis précédemment,
         qui peut être alkylé selon une technique classique d'alkylation pour conduire au composé de formule (I/m), cas particulier des composés de formule (I) : 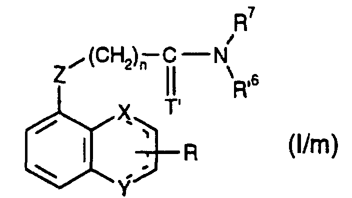 dans laquelle X, Y, Z, n, T', R et R⁷ sont tels que définis précédemment, et R'⁶ représente un groupement alkyle (C₁-C₆) linéaire ou ramifié,
      . ou par un azoture suivi d'une réduction pour conduire au composé de formule (III).
l'ensemble des composés (I/a) à (I/m) formant les composés de formule générale (I) qui peuvent être purifiés selon une technique classique de purification, dont on sépare, le cas échéant, les isomères selon une technique classique de séparation, que l'on transforme, si on le souhaite en leurs sels d'addition à un acide ou à une base pharmaceutiquement acceptable.

Les composés de formule (II) sont obtenus
à partir de composés de formule (X) : 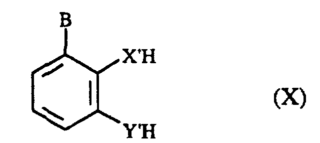 dans laquelle B représente un groupement méthyle, CHO, COOH, ou méthoxy, et X', Y', identiques ou différents représentent un atome d'oxygène ou de soufre,
sur lequel on condense le dibromoéthane pour conduire au composé de formule (XI): 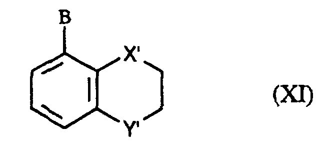 dans laquelle B, X' et Y' sont tels que définis précédemment,
composé (XI) que l'on soumet :
a) quand B représente un groupement méthyle à un agent de bromation comme le N-bromosuccinimide pour conduire au composé de formule (XII) : 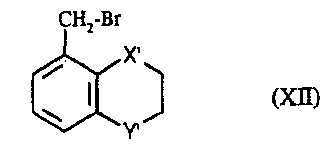 dans laquelle X' et Y' sont tels que définis précédemment.
b) quand B représente un groupement CHO à un ylure au phosphore de formule (XIII):

   (Ph₃)P = CH-(CH₂)ₚ-COOEt (XIII)

   dans laquelle p vaut 0, 1 ou 2,
   puis à une réduction, pour conduire au composé de formule (XIV): 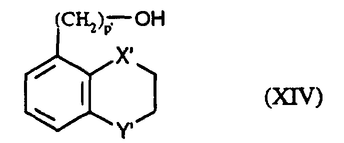 dans laquelle p' vaut 3, 4 ou 5 et X' et Y' sont tels que définis précédemment,
   qui est substitué par un brome pour conduire au composé de formule (XV), cas particulier des composés de formule (II) : 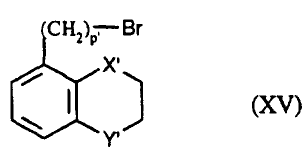 dans laquelle p', X' et Y' sont tels que définis précédemment,
c) quand B représente un groupement COOH, successivement à une estérification, une oxydation de l'hétérocycle. et une réduction de la fonction ester pour conduire au composé de formule (XVI): 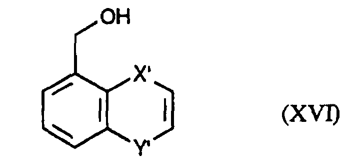 dans laquelle X' et Y' sont tels que définis précédemment,
   qui est soit substitué par un brome, soit oxydé en aldéhyde correspondant afin d'obtenir les homologues à chaîne plus longue,
d) quand B représente un groupement méthoxy, à une déméthylation suivi de la condensation d'un composé dihalogéné de formule (XVII) :

   Hal-(CH₂)ₙ-Hal' (XVII)

   dans laquelle n est tel que défini précédemment, et Hal et Hal' représentent un atome d'halogène,
   pour conduire au composé de formule (XVIII), cas particulier des composés de formule (II) : 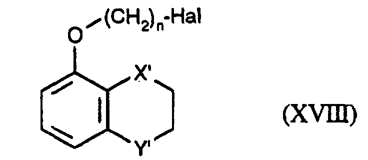 dans laquelle n. X', Y' et Hal sont tels que définis précédemment.

Les composés de formule (II) sont par ailleurs obtenus
- à partir du composé de formule (XIX) : 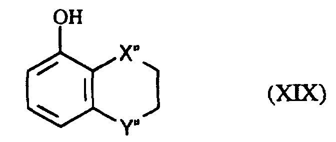
   dans laquelle X" représente un atome de soufre ou un atome d'oxygène et Y" représente un groupement CH₂,
   ou X" représente un groupement CH₂, et Y" représente un atome de soufre ou d'oxygène,
   sur lequel on condense un dérivé dihalogéné de formule (XVII) pour conduire au composé de formule (XX), cas particulier des composés de formule (II) : 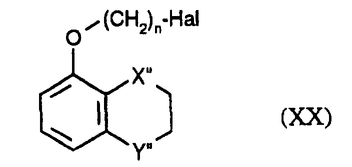 dans laquelle n, X", Y" et Hal sont tels que définis précédemment,
- ou à partir du composé de formule (XXI): 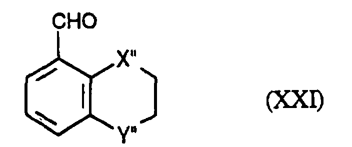 dans laquelle X" et Y" sont tels que définis précédemment,
qui est successivement soumis à une réaction de Wittig, une réduction puis à une substitution par un brome pour conduire au composé de formule (XXII), cas particulier des composés de formule (II) : 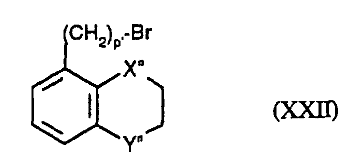 dans laquelle p', X" et Y" sont tels que définis précédemment,
la substitution sur le noyau hétérocyclique étant obtenue par des méthodes classiques sur le composé de formule (II) de départ ou sur le composé de formule (I).

Les composés de formule (I) possèdent des propriétés pharmacologiques très intéressantes pour le clinicien.

Les composés de l'invention et les compositions pharmaceutiques les contenant s'avèrent être utiles pour le traitement des troubles du système mélatoninergique.

L'étude pharmacologique des dérivés de l'invention a en effet montré qu'ils n'étaient pas toxiques, doués d'une très haute affinité sélective pour les récepteurs de la mélatonine et possédaient d'importantes activités sur le système nerveux central et en particulier, on a relevé des propriétés thérapeutiques sur les troubles du sommeil, des propriétés anxiolytiques, antipsychotiques, analgésiques ainsi que sur la microcirculation qui permettent d'établir que les produits de l'invention sont utiles dans le traitement du stress, des troubles du sommeil, de l'anxiété, des dépressions saisonnières, des pathologies cardiovasculaires, des insomnies et fatigues dues aux décalages horaires, de la schizophrénie, des attaques de panique, de la mélancolie, des troubles de l'appétit. de l'obésité, de l'insomnie, des troubles psychotiques, de l'épilepsie, du diabète, de la maladie de Parkinson, de la démence sénile, des divers désordres liés au vieillissement normal ou pathologique, de la migraine, des pertes de mémoire, de la maladie d'Alzheimer, ainsi que dans les troubles de la circulation cérébrale. Dans un autre domaine d'activité, il apparaît que dans le traitement, les produits de l'invention possèdent des propriétés d'inhibiteurs de l'ovulation, d'immunomodulateurs et qu'ils sont susceptibles d'être utilisés dans le traitement des cancers.

Les composés seront utilisés de préférence dans les traitements des dépressions saisonnières, des troubles du sommeil, des pathologies cardiovasculaires, des insomnies et fatigues dues aux décalages horaires, des troubles de l'appétit et de l'obésité.
Par exemple, les composés seront utilisés dans le traitement des dépressions saisonnières et des troubles du sommeil.
La présente invention a également pour objet les compositions pharmaceutiques contenant les produits de formule (I) en combinaison avec un ou plusieurs excipients pharmaceutiquement acceptables.

Parmi les compositions pharmaceutiques selon l'invention, on pourra citer, plus particulièrement celles qui conviennent pour l'administration orale, parentérale, nasale, per ou transcutanée, rectale, perlinguale, oculaire ou respiratoire et notamment les comprimés simples ou dragéifiés, les comprimés sublinguaux, les sachets, les paquets, les gélules, les glossettes, les tablettes, les suppositoires, les crèmes, les pommades, les gels dermiques, et les ampoules buvables ou injectables.

La posologie varie selon le sexe, l'âge et le poids du patient, la voie d'administration, la nature de l'indication thérapeutique, ou des traitements éventuellement associés et s'échelonne entre 0,01 mg et 1 g par 24 heures en 1 ou plusieurs prises.

Les exemples suivants illustrent l'invention, mais ne la limitent en aucune façon.

### Préparation 1 : 5-Bromométhyl-2,3-dihydro-1,4-benzodioxine

### Stade A : 5-Méthyl-2,3-dihydro-1,4-benzodioxine

A une solution d'orthométhyl catéchol (10 g) dissous dans 150 ml d'acétone sous atmosphère d'argon, est additionné le carbonate de potassium sec (0,93 éq.) ; le milieu réactionnel étant porté à un léger reflux, le dibromoéthane (0.4 éq.) est alors ajouté. Ces opérations sont renouvelées à deux reprises à 15 minutes d'intervalle et dans des proportions identiques (soit au total 2,8 éq. de K₂CO₃ et 1.2 éq. de dibromoéthane). Après 12 heures de reflux ce dernier est de nouveau introduit dans la réaction en quantité globalement identique (soit 1,2 éq.). Le chauffage est maintenu ainsi durant 72 heures. La solution obtenue étant refroidie, les sels sont filtrés sur célite, le filtrat quant à lui est concentré, hydrolysé par 60 ml d'eau puis extrait au dichlorométhane. La phase organique récupérée est lavée avec une solution de soude à 5%, séchée sur sulfate de magnésium, évaporée. Le produit cyclisé est purifié sur colonne de silice flash éluée par un mélange AcOEt/EP (2:8). Huile jaune.

### Stade B : 5-Bromométhyl-2,3-dihydro-1.4-benzodioxine

La bromation du 5-méthyl-2,3-dihydro-1,4-benzodioxine (4 g ; 27 mmol) est effectuée au sein du tétrachlorure de carbone anhydre (100 ml) sous atmosphère d'argon, le N-bromosuccinimide (4.98 g ; 28 mmol ; 1,05 éq.) ainsi qu'une pointe de spatule de 2.2'-azabisisobutyronitrile étant successivement introduits dans le milieu réactionnel. Ce dernier est porté à reflux durant 6 heures à l'aide d'une lampe (60 Watts). A température ambiante le succimide formé est filtré, le filtrat concentré est purifié sur une colonne de silice flash éluée à l'éther de pétrole puis à l'aide d'un mélange AcOEt/EP (1:9).
Point de fusion = 69°C

### Préparation 2 : 3-(2,3-Dihydro-1,4-benzodioxin-5-yl)-propionate d'éthyle

### Stade A : 5-Formyl-2,3-dihydro-1,4-benzodioxine

On procède comme pour le Stade A de la Préparation 1. Le produit cyclisé est purifié sur colonne de silice éluée par un mélange AcOEt/EP (3:7).
Point de fusion : 61°C

### Stade B : 5-(2-Carbéthoxyéthylène)-2,3-dihydro-1,4-benzodioxine

Le 5-formyl-2,3-dihydro-1,4-benzodioxine est mis en solution dans le toluène, et le carbéthoxyméthylènetriphénylphosphorane (2,2 éq.) est introduit. Le milieu réactionnel est porté à reflux durant 4 heures. L'oxyde de triphénylphosphine formé est alors précipité à froid en présence d'hexane. Le filtrat concentré est directement purifié sur une colonne de silice AcOEt/EP (1:9).
Stade C : 3-(2.3-Dihydro-1,4-benzodioxin-5-yl)-propionate d'éthyle

Le 5-(2-carbéthoxyéthylène)-2,3-dihydro-1,4-benzodioxine est solubilisé dans l'éthanol (20 ml) puis introduit dans le réacteur de l'appareil de Paar. Le palladium sur charbon à 10% (en masse) est alors additionné. La double liaison est hydrogénée sous pression de 45 psi durant 12 heures. Le catalyseur est éliminé par filtration puis le solvant est évaporé. L'huile résiduelle est purifiée sur une colonne de silice éluée au moyen d'un mélange AcOEt/EP (3:7). Huile incolore.

### Préparation 3 : 3-[(2,3-Dihydro-1,4-benzodioxin-5-yl)propyl]-4-méthyl-1-benzène sulfonate

### Stade A : 3-(2.3-Dihydro-1,4-benzodioxine-5-yl)-propanol

Une solution du composé obtenu dans la Préparation 2 (2.5 g ; 10,6 mmol) au sein de l'éther anhydre (20 ml) est injectée goutte à goutte sur l'hydrure de lithium et d'aluminium (401 mg ; 10,6 mmol ; 1 éq.) à 0°C. L'agitation est alors maintenue durant 1 heure à température ambiante. A 0°C le complexe formé est hydrolysé par des ajouts successifs de 0,32 ml d'eau, 0,32 ml d'une solution de soude à 15 %, 0,96 ml d'eau. Le mélange hétérogène est agité vivement durant 1 heure. Les sels ayant précipités, l'excès d'eau est éliminé à l'aide de sulfate de magnésium. Les résidus solides sont filtrés : le produit pur après concentration a l'aspect d'une huile incolore.

### Stade B : 3-[(2,3-Dihydro-1,4-benzodioxin-5-yl)propyl]-4-méthyl-1-benzène sulfonate

L'alcool obtenu au Stade A (800 mg : 4,12 mmol) étant solubilisé dans un mélange dichlorométhane/triéthylamine (20 ml/1,15 ml ; 8,24 mmol ; 2 éq.), le chlorure de tosyle (1,57 g : 8,24 mmol : 2 éq.) est alors ajouté. La tosylation est totale lorsque 12 heures d'agitation à température ambiante se sont écoulées. Après hydrolyse, les excès de réactif et de base sont éliminés par une extraction au dichlorométhane. La phase organique purifiée est concentrée par chromatographie sur gel de silice à l'aide d'un éluant binaire AcOEt/EP (3:7). Le produit tosylé a l'aspect d'une huile incolore.

### Préparation 4 : 3-(2,3-Dihydro-1,4-benzodioxin-5-yl)cyanopropane

Le déplacement du groupe tosyle du dérivé obtenu dans la Préparation 3 (2 g ; 5,74 mmol) s'effectue au sein du diméthylformamide à une température de 100°C durant 5 heures 30 en présence de cyanure de potassium (448 mg : 6,89 mmol ; 1,2 éq.). Après une légère hydrolyse à température ambiante le solvant est éliminé sous pression réduite puis le produit est extrait au dichlorométhane. La purification sur colonne de silice AcOEt/EP (3:7) de la phase organique concentrée permet l'obtention du composé du titre sous forme d'une huile jaune.

### Préparation 5 : 3-(1,4-Benzodioxin-5-yl)propylamine

### Stade A : 5-Carboxy-2,3-dihydro-1,4-benzodioxine

Un mélange de 10,8 g (70 mmol) d'acide 2,3-dihydroxybenzoïque, 38,6 g (280 mmol) de carbonate de potassium sec et de 24 ml (278 mmol) de 1,2-dibromoéthane dans 40 ml de N,N-diméthylformamide est chauffé à 65°C pendant 24 heures sous atmosphère inerte. Après refroidissement, le milieu réactionnel est dilué avec de l'eau puis extrait à l'éther. La phase aqueuse est ensuite acidifiée avec une solution d'acide chlorhydrique 3N puis extraite au dichlorométhane. Après évaporation du solvant sous vide, le résidu obtenu est recristallisé dans du toluène pour conduire à l'acide du titre sous forme d'un solide blanc.
Point de fusion : 193-194°C

### Stade B : 5-Carbométhoxy-2,3-dihydro-1,4-benzodioxine

3,46 g (19,2 mmol) de l'acide obtenu au Stade A sont ajoutés à un mélange de 9,62 g (114,6 mmol) d'hydrogénocarbonate de sodium et 3.57 ml (57,6 mmol) d'iodométhane dans 40 ml de N,N-diméthylacétamide. Après 24 heures d'agitation sous argon et à l'abri de la lumière, le solvant est évaporé sous vide. Le résidu obtenu est ensuite repris avec un mélange eau/acétate d'éthyle 1/1 puis la phase aqueuse est extraite à l'acétate d'éthyle. Après séchage sur sulfate de magnésium et filtration, le solvant est évaporé sous pression réduite. L'ester du titre est obtenu pur sous forme d'un solide blanc après une chromatographie sur gel de silice (éluant : AcOEt/EP : 25/75).
Point de fusion : 59°C

### Stade C : 5-Carbométhoxy-1,4-benzodioxine

2,58 g (13.3 mmol) d'ester obtenu au Stade B et 5,90 g (33,8 mmol) de N-bromosuccinimide en solution dans 40 ml de tétrachlorure de carbone anhydre sont portés à reflux après avoir ajouté une pointe de spatule d'AIBN. Après 9 heures, le mélange est laissé refroidir puis le succinimide formé est filtré. Le filtrat est concentré sous pression réduite pour conduire quantitativement à l'ester dibromé. Cet ester est ensuite repris dans 40 ml d'acétone anhydre puis 7 g d'iodure de sodium (46,5 mmol) sont ensuite ajoutés à la solution. Après 4 jours d'agitation à température ambiante et sous atmosphère inerte, le solvant est évaporé sous vide puis le résidu obtenu est repris dans un mélange eau/acétate d'éthyle 1/1. La phase aqueuse est extraite à l'acétate d'éthyle. Les phases organiques sont décolorées à l'aide d'une solution de thiosulfate de sodium saturée puis séchées sur sulfate de magnésium. Après évaporation du solvant sous pression réduite, l'ester du titre est obtenu pur sous forme d'un sirop brun après passage sur colonne de silice (éluant : AcOEt/EP : 15/85) avec un rendement global de 77%.

### Stade D : 5-Hydroxyméthyl-1,4-benzodioxine

1,62 g (8,44 mmol) de l'ester insaturé obtenu au Stade C sont ajoutés à une suspension de 0.64 g (16,84 mmol) d'hydrure de lithium aluminium dans 40 ml d'éther anhydre, le milieu est ensuite porté à reflux pendant 30 minutes sous atmosphère inerte puis laissé refroidir. La solution est hydrolysée avec, successivement. 0,64 ml d'eau, 0,64 ml d'une solution de soude à 15 % et enfin 1,92 ml d'eau. Après 30 minutes d'agitation, les sels sont filtrés puis le filtrat est concentré sous pression réduite. L'alcool du titre est obtenu pur sous forme d'un solide blanc après une chromatographie sur gel de silice (éluant : AcOEt/EP : 40/60).
Point de fusion : 57°C

### Stade E : 5-Formyl- 1,4-benzodioxine

Une solution de 1,27 g (10,05 mmol) de chlorure d'oxalyle dans 30 ml de dichlorométhane anhydre est refroidie à -60°C puis 1,57 g (20,1 mmol) de diméthylsulfoxyde sont ajoutés au milieu. Après 5 minutes d'agitation sous atmosphère inerte, 1.1 g de 5-hydroxyméthyl-1,4-benzodioxine (6,7 mmol) en solution dans 15 ml de dichlorométhane sont lentement additionnés au milieu puis le mélange est agité pendant 15 minutes supplémentaires. 4,66 ml (33.5 mmol) de triéthylamine sont alors ajoutés au milieu qui est laissé, ensuite, revenir à température ambiante. La solution est alors acidifiée à l'aide d'une solution d'acide chlorhydrique (1N) puis la phase aqueuse est extraite au dichlorométhane. La phase organique, séchée sur sulfate de magnésium puis filtrée, est concentrée sous pression réduite. L'aldéhyde du titre est obtenu pur sous forme d'un solide jaune après passage sur colonne de silice (éluant : AcOEt/EP : 10/90).
Point de fusion : 54°C

### Stade F : (E+Z)-3-(1,4-Benzodioxin-5-yl)acrylonitrile

1.11 ml (6.87 mmol) de cyanométhylphosphonate de diéthyle sont ajoutés à une suspension de 0,28 g (6,9 mmol à 60%) d'hydrure de sodium dans 20 ml de tétrahydrofurane anhydre préalablement refroidie à 0°C. Après 10 minutes d'agitation sous argon, la température est abaissée à -78°C puis 0,97 g (6 mmol) de 5-formyl-1,4-benzodioxine en solution dans 20 ml de tétrahydrofurane anhydre est lentement additionné au milieu réactionnel. Après 90 minutes à -78°C, le mélange est laissé revenir à température ambiante puis hydrolysé avec une solution d'hydrogénocarbonate de sodium saturée. La phase aqueuse est extraite à l'acétate d'éthyle puis la phase organique est séchée sur sulfate de magnésium. Le solvant est ensuite évaporé sous pression réduite puis le résidu obtenu est purifié sur colonne de silice (éluant : AcOEt/EP : 30/70) pour conduire au nitrile du titre sous forme d'un solide jaune.
Point de fusion : 102-103°C

### Stade G : 3-(1,4-Benzodioxin-5-yl)propylamine

0,925 g (5 mmol) du nitrile obtenu au Stade F est dissous dans 40 ml d'éther anhydre puis 0.76 g (20 mmol) d'hydrure de lithium aluminium sont ajoutés en plusieurs portions au milieu réactionnel. Le mélange réactionnel est porté à reflux pendant 5 heures sous argon puis laissé revenir à température ambiante. L'hydrolyse est ensuite effectuée selon la procédure habituelle. Après filtration des sels et évaporation du solvant sous vide, l'amine du titre est obtenue pure sous forme d'une huile jaune.

### Préparation 6 : 5-Hydroxy-2,3-dihydro-1,4-benzodioxine

### Stade A : 5-Méthoxy-2.3-dihydro-1,4-benzodioxine

On procède comme pour le Stade A de la préparation 1. Le dérivé cyclisé est purifié par une colonne de silice flash éluée par un mélange AcOEt/EP (2:8). Huile jaune.

### Stade B : 5-Hydroxy-2,3-dihydro-1,4-benzodioxine

A une température de -10°C, le tribromure de bore (11,34 ml ; 120 mmol ; 2éq.) est injecté lentement dans un mélange anhydre du composé obtenu dans le Stade A (10 g : 60 mmol) dans le dichlorométhane (100 ml). Le milieu est agité ainsi durant 2 heures 30. L'hydrolyse est effectuée à 0°C à l'aide d'une solution saturée d'hydrogénocarbonate de sodium ; le produit est extrait à un pH égal à 8. La phase organique séchée sur sulfate de magnésium est concentrée puis purifiée par flash chromatographie, l'éluant binaire étant composé d'acétate d'éthyle et d'éther de pétrole en proportions 3:7. Huile jaune.

### Préparation 7 : 2-(2,3-Dihydro-1,4-benzodioxin-5-yloxy)-éthylamine

### Stade A : 5-(2-Bromoéthoxy)-2,3-dihydro-1,4-benzodioxine

Le carbonate de potassium sec (10 éq.) est ajouté à une solution du dérivé phénolique obtenu dans la Préparation 6 (2 g) dans le toluène (100 ml). Lorsque 30 minutes de chauffage à reflux se sont écoulées, le dibromoéthane (5 éq.) ainsi que l'agent de transfert de phase, le bromure de tétrabutylammonium (0,2 éq.) sont additionnés. Après 24 heures de reflux, les sels sont filtrés sur célite et le toluène éliminé sous pression réduite. Le dérivé obtenu est extrait au dichlorométhane en milieu légèrement basique (solution de soude à 5%). La phase organique concentrée est purifiée sur colonne de silice normale éluée par un mélange AcOEt / EP (3:7).
Point de fusion : 85°C

### Stade B : 2-[2-(2.3-Dihydro-1,4-benzodioxin-5-yl)-éthoxy]-isoindole-1,3-dione

La substitution du brome du composé obtenu au Stade A s'effectue dans le diméthylformamide (10 ml) sous atmosphère d'argon en présence de phthalimidure de potassium (1,5 éq.) et ce, sous catalyse par l'iodure de potassium (0,07 éq.). Le milieu réactionnel est agité et porté à reflux durant 3 heures. Les sels restants et résultants sont éliminés par filtration : le produit est alors précipité à froid par addition d'eau dans le filtrat. Le solide formé est filtré puis séché sous vide en présence de pentoxyde de phosphore.
Point de fusion : 156°C

### Stade C : 2-(2,3-Dihydro-1,4-benzodioxin-5-yloxy) éthylamine

La coupure du groupement phtalimide du composé obtenu au Stade B s'effectue dans l'éthanol en présence d'hydrate d'hydrazine en solution aqueuse à 98% (2 éq. pour 1,5 g de produit). Il est préférable d'additionner cette dernière après 10 minutes de reflux. Lorsque la réaction est terminée, l'alcool est éliminé sous pression réduite. Le résidu sec est repris dans le dichlorométhane à froid afin de faire précipiter le phtalhydrazide formé. Ce dernier peut alors être éliminé par filtration, le filtrat récupéré et concentré est lavé ainsi à plusieurs reprises jusqu'à l'obtention du produit pur en chromatographie sur couche mince.
Point de fusion : 65°C

### Préparation 8 : 8-(2-Bromoéthoxy)-3,4-dihydro-2H-1-benzothiopyrane

Le bromure de tétrabutylammonium (1,94 g ; 6 mmol ; 0,2 éq.), agent de transfert de phase, est ajouté à un mélange hétérogène composé du 8-hydroxy-3,4-dihydro-2*H*-1-benzothiopyrane (5 g ; 30 mmol), d'acétonitrile (30 ml) et d'une solution de soude 1,6 N (28,3 ml ; 45 mmol ; 1,5 éq.). Le dibromoéthane (10ml ; 60 mmol ; 1,5 éq.) est additionné après 30 minutes d'agitation. Cette dernière est maintenue durant 24 heures à 30°C. La concentration des diverses phases sous pression réduite à 25°C précède l'extraction des produits au dichlorométhane. Un lavage à l'aide d'une solution de soude à 5% permet une première purification qui, poursuivie par une colonne de silice éluée par un mélange AcOEt/EP (3:7), conduit au dérivé bromé du titre.
Point de fusion : 62°C

### Préparation 9 : 5-(3-Bromopropyl)-2,3-dihydro-1,4-benzoxathiine

Une solution décimolaire du 8-(2-bromoéthoxy)-3,4-dihydro-2*H*-1-benzothiopyrane obtenu dans la Préparation 8 (546 mg) dans 20 ml d'acétonitrile est portée à reflux durant 48 heures. Le solvant étant éliminé, le brut réactionnel est purifié par une colonne de silice éluée par un gradient EP / CH₂Cl₂ allant progressivement jusqu'à un mélange 2:1. Le produit du titre est obtenu pur sous forme d'une huile incolore.

### Préparation 10: 5-(3-Cyanopropyl)-2,3-dihydro-1,4-benzoxathiine

La substitution du brome du composé de la Préparation 9 (200 mg) par le cyanure de potassium (1,1 éq. dans un premier temps) s'effectue à température ambiante au sein de diméthylformamide (5 ml) sous atmosphère inerte. Après 12 heures d'agitation un ajout similaire de réactif (1,1 éq.) permet de relancer la substitution. Après hydrolyse, le solvant est éliminé sous pression réduite. L'extraction du produit se fait au dichlorométhane : le concentrat résultant des phases organiques est purifié sur gel de silice élué par un mélange AcOEt/EP (3:7). Huile incolore.

### Préparation 11 : Acide 4-(2,3-dihydro-1,4-benzoxathiin-5-yl)-butanoïque

Le dérivé cyané obtenu dans la Préparation 10 en solution dans l'éthanol (10 ml) est alcalinisé à l'aide d'une solution de soude à 10% (5 éq.) et ce, durant 24 heures à 60°C. L'alcool est éliminé sous vide en totalité, les traces résiduelles de substrats étant quant à elles extraites à l'acétate d'éthyle. L'acide est précipité par acidification (solution d'acide chlorhydrique 2N) de la phase aqueuse, la filtration de cette dernière donne le produit pur, l'eau disparaissant par séchage sous vide en présence de pentoxyde de phosphore.
Point de fusion : 93°C

### Préparation 12 : 3-(2,3-Dihydro-1,4-benzoxathiin-5-yl)-propanal

### Stade A : 5-(3-Acétoxypropyl)-2.3-dihydro-1,4-benzoxathiine

L'acétate mercurique (1.17 g ; 3.66 mmol ; 1 éq.) est introduit dans une solution du dérivé bromé obtenu dans la Préparation 9 (1 g : 3,66 mmol) dans l'acide acétique glacial (20 ml). Le solvant étant à reflux, le chauffage est maintenu durant 4 heures. Le milieu réactionnel concentré est repris dans l'acétate d'éthyle afin d'effectuer une filtration permettant l'élimination des sels. Le filtrat peut alors être extrait puis lavé avec une solution saturée d'hydrogénocarbonate de sodium. La phase organique concentrée est purifié sur une colonne de silice AcOEt/EP (1:3). Les dernières traces de réactif sont éliminées par un lavage à l'aide d'un mélange binaire dichlorométhane/cyclohexane (1:1); l'ester pur est une huile jaune.

### Stade B : 3-(2.3-Dihydro-1,4-benzoxathiin-5-yl)-propanol

La coupure de l'ester obtenu au Stade A (1,2 g : 4,75 mmol) est réalisée par le carbonate de potassium (986 mg : 7,13 mmol ; 1.5 éq.) au sein d'un mélange MeOH/H₂O (48 ml : 12 ml). Le groupement acétate est clivé après 2 heures 30 minutes d'agitation à température ambiante. Les sels étant filtrés, les solvants concentrés, le produit est extrait à l'acétate d'éthyle. Une filtration sur colonne de silice flash (CH₂Cl₂ 100%) de l'huile résiduelle permet d'isoler le composé sous forme d'une huile de couleur jaune.

### Stade C : 3-(2.3-Dihydro-1,4-benzoxathiin-5-yl)-propanal

Une solution de chlorure d'oxalyle (0,68 ml ; 7,85 mmol ; 1,1 éq.) dans le dichlorométhane anhydre (18,8 ml) est préparée sous argon. A cette dernière est ajouté le diméthylsulfoxyde distillé dans 3.7 ml de dichlorométhane à -50°C. Le milieu réactionnel est laissé ainsi 2 minutes puis l'alcool obtenu au Stade B (1,5 g ; 7,13 mmol) en solution dans 7,5 ml de dichlorométhane est lentement transféré. Après 30 minutes, est additionnée à froid de la triéthylamine distillée (4,97 ml ; 35,7 mmol ; 5 éq.). La température est remontée doucement après 10 minutes, l'agitation étant alors maintenue 2 heures. Le produit est extrait au dichlorométhane, lavé abondamment à l'eau puis à l'aide d'une solution saturée de chlorure de sodium. L'huile brute purifiée sur une colonne de silice AcOEt/EP (3:7) présente à l'état pur une couleur jaune.

### EXEMPLE 1 : N-[(2,3-Dihydro-1,4-benzodioxin-5-yl)-méthyl]-acétamide

### Stade A : 2-[(2,3-Dihydro-1,4-benzodioxin-5-yl)-méthyl]-isoindole-1,3-dione

On procède comme dans le Stade B de la Préparation 7 à partir du produit de la Préparation 1.
Point de fusion : 204°C

### Stade B : N-[(2,3-Dihydro-1,4-benzodioxin-5-yl)-méthyl]-méthanamine

On procède comme dans le Stade C de la Préparation 7 à partir du composé obtenu dans le Stade A. Huile jaune.

### Stade C : N-[(2.3-Dihydro-1.4-benzodioxin-5-yl)-méthyl]-acétamide

L'amine obtenue au Stade B est solubilisée dans la pyridine (5 ml) sous atmosphère d'argon, et l'anhydride acétique (1,2 éq.) est additionné 0°C. L'agitation est poursuivie à température ambiante durant 2 heures, l'acétylation est alors totale. Le solvant étant éliminé sous pression réduite, le milieu réactionnel est extrait au dichlorométhane. La phase organique est lavée à l'eau, séchée sur sulfate de magnésium puis concentrée. Le produit ainsi obtenu est séché sous vide afin d'éliminer les traces résiduelles de pyridine avant la purification : cette dernière s'effectue par flash chromatographie à l'aide d'un éluant composé d'un mélange CHCl₃/AcOEt (7:3).
Point de fusion : 118°C

### EXEMPLE 2 : N-[(2,3-Dihydro-1,4-benzodioxin-5-yl)-méthyl]-N-méthyl acétamide

Le composé du titre est obtenu par alkylation classique du composé obtenu dans l'Exemple 1 en milieu basique à l'aide de diméthylsulfate.

### EXEMPLE 3 : N-[2-(2,3-Dihydro-1,4-benzodioxin-5-yl)-éthyl]-acétamide

### Stade A : 5-Cyanométhyl-2.3-dihydro-1.4-benzodioxine

On procède comme pour la Préparation 10, à partir du composé de la Préparation 1. La purification est effectuée au moyen d'un mélange AcOEt / EP (1:9).
Point de fusion : 47°C

### Stade B : 2-(2,3-Dihydro-L4-benzodioxin-5-yl) éthylamine

Une solution du dérivé cyané obtenu au Stade A (1,5 g ; 8,37 mmol) dans l'éther anhydre (25 ml) est ajoutée à l'hydrure de lithium et d'aluminium (635 mg ; 16,7 mmol ; 2 éq.) à 0°C. Le tout est ensuite porté à reflux pendant 4 heures. Après refroidissement, le complexe est hydrolysé successivement par 0,63 ml d'eau, 0,63 ml de solution de soude à 15% et 1,89 ml d'eau. Les sels d'alumine précipitent finalement au bout d'une heure d'agitation. Les traces aqueuses sont éliminées à l'aide de sulfate de magnésium ajouté au milieu réactionnel. Le filtrat obtenu par filtration est alors concentré donnant ainsi l'amine pure sous forme d'une huile jaune.

### Stade C : N-[2-(2,3-Dihydro-1,4-benzodioxin-5-yl)-éthyl]-acétamide

On procède comme dans le Stade C de l'Exemple 1 à partir du composé obtenu au Stade B.
Point de fusion : 57°C

### EXEMPLE 4 : N-Méthyl-[3-(2,3-dihydro-1,4-benzodioxin-5-yl)]-propanamide

### Stade A : Acide -3-(2,3-dihydro-1,4-benzodioxin-5-yl)-propanoïque

Une solution de soude à 10% (2 éq.) est introduite sous atmosphère d'argon dans un milieu réactionnel comportant l'ester obtenu dans la Préparation 2 solubilisé dans l'éthanol (10 à 15 ml). Après deux heures de chauffage à reflux, on extrait à l'acétate d'éthyle. L'acidification à froid de la phase aqueuse par une solution d'acide chlorhydrique 2N puis 3N entraîne la précipitation du produit attendu. Ce dernier est filtré puis séché sous vide en présence de pentoxyde de phosphore.
Point de fusion : 73°C

### Stade B : N-Methyl-[3-(2,3-dihydro-1,4-benzodioxin-5-yl)]-propanamide

Les agents de couplage tels que l'hydroxybenzotriazole (1,1 éq.) et le chlorhydrate du 1-(3-diméthylaminopropyl)-3-éthylcarbodiimide (1,1 éq.) sont introduits à 0°C dans un milieu réactionnel comprenant l'acide obtenu au Stade A en solution dans le diméthylformamide anhydre (10 ml) sous atmosphère inerte. La circulation d'argon étant interrompue la méthylamine en solution à 10% dans le benzène (1,5 éq.) peut alors être additionnée. La température est ramenée très lentement à 20°C, l'agitation est maintenue ainsi durant 18 heures. L'évaporation du solvant permet l'extraction du produit par le dichlorométhane, un lavage à l'eau éliminant les excès de réactifs. La phase organique séchée, concentrée est purifiée par flash chromatographie éluée à l'aide d'un mélange CHCl₃/AcOEt (7:3).
Point de fusion : 99°C

### EXEMPLE 5 : N-Méthyl-[3-(2,3-Dihydro-1,4-benzodioxin-5-yl)]-propanethioamide

Le composé du titre est obtenu par action du réactif de Lawesson sur le composé obtenu dans l'Exemple 4.

### EXEMPLE 6 : N-[4-(2,3-Dihydro-1,4-benzodioxin-5-yl)-butyl]-acétamide

### Stade A : 4-(2,3-Dihydro-1,4-benzodioxin-5-yl) butylamine

On procède comme dans le Stade B de l'Exemple 3 à partir du composé de la Préparation 4.

### Stade B : N-[4-(2.3-Dihydro-1,4-benzodioxin-5-yl)-butyl]-acétamide

On procède comme dans le Stade C de l'Exemple 3 à partir du composé obtenu dans le Stade A. Gomme jaune.

### EXEMPLE 7 : N-[4-(2,3-Dihydro-1,4-benzodioxin-5-yl)butyl]-3-butènamide

En procédant comme dans l'Exemple 6 en utilisant l'anhydride approprié dans le Stade B, on obtient le composé de l'Exemple 7.

### EXEMPLE 8 : N-[3-(2,3-Dihydro-1,4-benzodioxin-5-yl)-propyl]-acétamide

### Stade A : 5-(3-Azidopropyl)-2,3-dihydro-1.4-benzodioxine

La substitution du groupement tosyle du dérivé obtenu dans la Préparation 3 (1 g ; 2,87 mmol) est effectuée au sein du diméthylformamide (30 ml) en présence d'azoture de sodium (560 mg : 8.61 mmol ; 3 éq.). La réaction est totale après 12 heures d'agitation à température ambiante. Le solvant étant éliminé. le produit est extrait au dichlorométhane, lavé à l'eau puis purifié sur colonne de silice éluée par un mélange AcOEt/EP (3:7). L'azoture recherché se présente sous forme d'une huile incolore.

### Stade B : N-[3-(2,3-Dihydro-1,4-benzodioxin-5-yl)-propyl]-acétamide

L'amine est tout d'abord synthétisée par hydrogénation catalytique du composé obtenu au Stade A. Ce dernier (900 mg ; 4.1 mmol) est solubilisé à l'aide d'éthanol (15 ml) dans le réacteur de l'appareil de Paar. Après l'ajout du catalyseur en l'occurrence le palladium sur charbon à 10% (90 mg ; 10% en masse), l'agitation est maintenue durant 4 heures à température ambiante à une pression d'hydrogène de 45 psi. Le milieu réactionnel étant filtré puis concentré sous pression réduite, l'amine est récupérée sous forme d'une huile incolore. Cette dernière représente une pureté correcte en chromatographie sur couche mince et sera donc acétylée directement dans les conditions du Stade C de l'Exemple 1. Huile jaune pâle.

### EXEMPLE 9 : N-[3-(2,3-Dihydro-1,4-benzodioxin-5-yl)propyl]-2-phénylacétamide

On procède comme dans l'Exemple 8 en remplaçant l'anhydride acétique par l'anhydride phénylacétique.

De même en rempaçant l'anhydride acétique par l'anhydride cyclohexane carboxylique, on obtient le composé de l'exemple 10 :

### EXEMPLE 10 : N-[3-(2,3-Dihydro-1,4-benzodioxin-5-yl)propyl]cyclohexnne carboxamide

### EXEMPLE 11 : N-Méthyl-[4-(2,3-dihydro-1,4-benzodioxin-5yl)]-butanamide

### Stade A : Acide-4-(2,3-dihydro-1,4-benzodioxin-5-yl)-butanoïque

On procède de la même façon que dans la Préparation 11, à partir du composé obtenu dans la Préparation 4.
Point de fusion : 54°C

### Stade B : N-Méthyl-[4-(2,3-dihydro-1.4-benzodioxin-5-yl)]-butanamide

On procède comme dans le Stade B de l'Exemple 4 à partir de l'acide-4-(2,3-dihydro-1,4-benzodioxin-5-yl)-butanoïque.
Point de fusion : 79°C

| | | | |
|---|---|---|---|
| Microanalyse Elémentaire : | C | H | N |
| % Calculé | 66,36 | 7,28 | 5,95 |
| % Trouvé | 66,31 | 7,21 | 5,95 |

### EXEMPLE 12 : N-Cyclobutyl-4-(2,3-Dihydro-1,4-benzodioxin-5-yl)butanamide

On procède comme dans l'Exemple 11 en remplaçant la méthylamine par la cyclobutylamine.

### EXEMPLE 13 : N-Méthyl-[5-(2,3-dihydro-1,4-benzodioxin-5-yl)]-pentanamide

### Stade A : 3-(2,3-Dihydro-1,4-benzodioxin-5-yl)-propanal

L'hydrure de diisobutylaluminium (1M) (3,43 ml ; 3,43 mmol ; 0,9 éq.) est injecté lentement à -78°C dans une solution de l'ester obtenu dans la Préparation 2 dissous dans le toluène. Après 45 mn d'agitation à une température identique, l'hydrolyse est effectuée à l'aide d'acide chlorhydrique 2N. A 20-25°C, les produits sont extraits à l'AcOEt puis purifiés sur colonne de silice éluée avec un mélange AcOEt/EP (2:8). Le composé recueilli sous forme d'une huile incolore correspond à l'aldéhyde.

### Stade B : 5-(4-Carbéthoxy-but-3-ène)-2,3-dihydro-1,4-benzodioxine

On procède comme dans le Stade B de la Préparation 2 à partir du composé obtenu au Stade A. Huile incolore.

### Stade C : 5-(2,3-Dihydro-1,4-benzodioxin-5-yl)-pentanoate d'éthyle

L'hydrogénation catalytique de l'insaturation de l'ester obtenu au Stade B (600 mg ; 2.29 mmol) aboutit à une huile incolore présentant l'enchaînement butyle caractéristique du dérivé souhaité.

### Stade D : Acide-5-(2,3-dihydro-1.4-benzodioxin-5-yl)-pentanoïque

On procède comme dans le Stade A de l'Exemple 4, à partir de l'ester obtenu au Stade C.
Point de fusion : 56°C

### Stade E : N-Méthyl-[5-(2.3-dihydro-1,4-benzodioxin-5-yl)]-pentanamide

On procède comme dans le Stade B de l'Exemple 4, à partir de l'acide obtenu au Stade D.
Point de fusion : 79°C

| | | | |
|---|---|---|---|
| Microanalyse Elémentaire : | C | H | N |
| % Calculé | 67,45 | 7,68 | 5,62 |
| % Trouvé | 67,50 | 7,70 | 5,41 |

### EXEMPLE 14 : N-Benzyl-5-(2,3-dihydro-1,4-benzodioxin-5-yl)pentanamide

On procède comme dans l'Exemple 13 en remplaçant la N-méthyl amine par la N-Benzylamine.

### EXEMPLE 15 : N-[3-(1,4-Benzodioxin-5-yl)-propyl]-acétamide

Une solution de 0.885 g (4,63 mmol) de l'amine obtenue dans la Préparation 5 dans 10 ml de pyridine anhydre est refroidie à 0°C puis 0,53 ml (5,56 mmol) d'anhydride acétique sont ajoutés au milieu. Après une heure d'agitation à 0°C et sous atmosphère inerte, le milieu est dilué par de l'acétate d'éthyle puis la phase organique est acidifiée avec une solution d'acide chlorhydrique (1N). Après extraction de la phase aqueuse à l'acétate d'éthyle, les phases organiques sont séchées sur sulfate de magnésium puis concentrées sous pression réduite. Le résidu obtenu est purifié sur colonne de silice (éluant : méthanol/dichlorométhane 3:97) pour conduire à l'amide du titre sous forme d'un sirop clair.

### EXEMPLE 16 : N-[3-(3-Méthyl-1,4-benzodioxin-5-yl)propyl]acétamide

### EXEMPLE 17 : N-[3-(3-Benzyl-1,4-benzodioxin-5-yl)propyl]acétamide

Les exemples 16 et 17 sont obtenus comme suit :

Après protection de l'amine obtenue dans la Préparation 5 sous forme de dibenzylamine, on procède à une lithation grâce au disopropyl amidure de Lithium puis à la condensation de l'électrophile désiré. Après débenzylation par hydrogénation catalytique et acétylation selon la procédure utilisée dans le Stade C de l'Exemple 1, on obtient les composés du titre.

### EXEMPLE 18 : N-[3-(3-Méthyl-2,3-dihydro-1,4-benzodioxin-5-yl)propyl]acétamide

On procède comme pour l'Exemple 16 en utilisant des conditions de température et de pression plus fortes lors de l'hydrogénation catalytique.

### EXEMPLE 19 : N-[3-(1,4-Benzodioxin-5-yl)-propyl]-butanamide

Une solution de 1 g (5,23 mmol) de l'amine obtenue dans la Préparation 5 dans 20 ml de dichlorométhane anhydre est refroidie à 0°C puis 0,78 g (7,32 mmol) de chlorure de butyryle ainsi que 1,58 g (15,7 mmol) de triéthylamine sont successivement additionnés au milieu.

Après 30 minutes d'agitation, le mélange réactionnel est acidifié avec une solution d'acide chlorhydrique (1N). Après extraction à l'aide du dichlorométhane, les phases organiques sont séchées sur sulfate de magnésium puis concentrées sous vide. Le résidu obtenu est purifié sur colonne de silice (éluant : éther de pétrole/acétate d'éthyle 50:50) pour aboutir à l'amide du titre sous forme d'une huile dense.

### EXEMPLE 20 : N-[2-(2,3-Dihydro-1,4-benzodioxin-5-yloxy)-éthyl]-acétamide

On utilise la même procédure que dans le Stade C de l'Exemple 1 à partir du composé de la Préparation 7.

### EXEMPLE 21 : N-[2-(2,3-Dihydro-1,4-benzodioxin-5-yloxy)éthyl]-2-phényl acétamide

On procède comme dans l'Exemple 20 en remplaçant l'anhydride acétique par l'anhydride phénylacétique.

### EXEMPLE 22 : N-[2-(2,3-Dihydro-1,4-benzodioxin-5-yloxy)-éthyl]-cyclopropanecarboxamide

Le chlorure de cyclopropanoyle (0,74 ml ; 8,2 mmol ; 1,6 éq.) est introduit lentement à un milieu réactionnel comprenant l'amine obtenue dans la Préparation 7 (1 g ; 5,1 mmol) en solution dans un mélange dichlorométhane/triéthylamine (20 ml ; 9,28 ml ; 67 mmol ; 13 éq.). Cette opération s'effectuant à 0°C, la réaction est ramenée doucement à température ambiante durant 2 heures d'agitation. Les excès de réactif et de base sont éliminés par des lavages à l'eau lors de l'extraction au dichlorométhane. La phase organique séchée, concentrée conduit à un solide orange qui est purifié sur colonne de silice (CH₂Cl₂/MeOH : 2%) puis recristallisé dans un mélange binaire éther diéthylique/acétonitrile.
Point de fusion : 137°C

### EXEMPLE 23 : N-[2-(2,3-Dihydro-1,4-benzodioxin-5-yloxy)éthyl]-N'-propylurée

Après dissolution de l'amine obtenue dans la Préparation 7 (800 mg ; 4,1 mmol) dans le toluène sous atmosphère d'argon, le n-propyl isocyanate (0,58 ml ; 6,15 mmol ; 1,5 éq.) est ajouté à 0°C. Le milieu réactionnel est laissé sous agitation à température ambiante durant 2 heures. L'excès de réactif est alors neutralisé par hydrolyse, l'élimination du solvant sous pression réduite conduit à un mélange qui est extrait au dichlorométhane. La purification fait appel d'une part à une colonne sur silice éluée par un mélange CH₂Cl₂/MeOH 2% et d'autre part à un lavage à l'éther diéthylique.
Point de fusion : 164°C

### EXEMPLE 24 : N-[2-(2,3-Dihydro-1,4-benzodioxin-5-yl)éthyl]-N'-propylurée

On procède comme dans l'Exemple 23 à partir de l'amine obtenue dans le Stade B de l'Exemple 3.

### EXEMPLE 25 : N-[4(2,3-Dihydro-1,4-benzodioxin-5-yloxy)-butyl]-acétamide

### Stade A : 5-(4-Bromobutoxy)-2,3-dihydro-1,4-benzodioxine

On utilise la même procédure que dans le Stade A de la Préparation 7 à partir du composé de la Préparation 6.
Point de fusion : 42°C

### Stade B : 2-[4-(2,3-Dihydro-1,4-benzodioxin-5-yloxy)-butyl]-isoindole-1,3-dione

On utilise le même procédé que dans le Stade B de la Préparation 7 à partir du composé obtenu au Stade A.
Point de fusion : 112°C

### Stade C : 4-(2,3-Dihydro-1,4-benzodioxin-5-yloxy) butylamine

On utilise le même procédé que dans le Stade C de la Préparation 7 à partir du composé obtenu au Stade B. Huile jaune.

### Stade D : N-[4-(2,3-Dihydro-1,4-benzodioxin-5-yloxy)-butyl]-acétamide

On utilise le même procédé que dans le Stade C de l'Exemple 1 à partir du composé obtenu au Stade C.
Point de fusion : 91°C

| Microanalyse Elémentaire : | C | H | N |
|---|---|---|---|
| % Calculé | 63,38 | 7,22 | 5,28 |
| % Trouvé | 63,20 | 7,22 | 5,30 |

De façon analogue à l'Exemple 25, on obtient le composé de l'Exemple 26.

### EXEMPLE 26 : N-[3-(2,3-Dihydro-1,4-benzodioxin-5-yloxy)-propyl]-acétamide

### Stade A : 5-(3-Bromopropoxy)-2,3-dihydro-1,4-benzodioxine

Point de fusion : 54°C

### Stade B : 2-[3-(2,3-Dihydro-1,4-benzodioxin-5-yloxy)-propyl]-isoindole-1,3-dione

Point de fusion : 128°C

### Stade C : 3-(2,3-Dihydro-1,4-benzodioxin-5-yloxy) propylamine

Point de fusion : 134°C

### Stade D : N-[3-(2,3-Dihydro-1,4-benzodioxin-5-yloxy)-propyl]-acétamide

Point de fusion : 96°C

### EXEMPLE 27 : N-[3-(2,3-Dihydro-1,4-benzodioxin-5-yloxy)-propyl]-éthanethioamide

Le produit du titre est obtenu en traitant le composé obtenu dans l'Exemple 26 par le réactif de Lawesson.

### EXEMPLE 28 : N-Méthyl-[3-(4,4-dioxo-2,3-dihydro-1,4-benzoxathiin-5-yl)]-propanamide

### Stade A : Acide 3-(4,4-dioxo-2,3-dihydro-1,4-benzoxathiin-5-yl)-propanoïque

150 mg du composé obtenu dans la Préparation 12 sont dissous dans le tertbutanol (5 ml) et une solution molaire de permanganate de potassium (4,32 ml ; 4,32 mmol : 6 éq.) ainsi qu'une solution tampon d'hydrogénophosphate 0,16 M (0,9 ml ; 0,144 mmol ; 0,2 éq.) sont introduites. Après 40 minutes d'agitation à température ambiante, l'excès de permanganate de potassium est traité par une solution saturée au sulfite de sodium. L'oxyde de manganèse formé étant filtré, le filtrat concentré est extrait au dichlorométhane. La précipitation de la phase aqueuse en milieu acide permet d'accéder à l'acide.

### Stade B : N-Méthyl-[3-(4,4-dioxo-2,3-dihydro-1.4-benzoxathiin-5-yl)]-propanamide

On utilise le même procédé que dans le Stade B de l'Exemple 4 à partir de l'acide obtenu dans le Stade A.
Point de fusion : 157°C

| Microanalyse Elémentaire : | C | H | N | S |
|---|---|---|---|---|
| % Calculé | 53,52 | 5,61 | 5,20 | 11,91 |
| % Trouvé | 53,56 | 5,57 | 5,27 | 11,91 |

### EXEMPLE 29 : N-[5-(2,3-dihydro-1,4-benzoxathiin-5-yl)-pentyl]-acétamide

### Stade A : 5-(4-Cyano-but-3-ène)-2,3-dihydro-1,4-benzoxathiine

On procède comme au Stade F de la Préparation 5 à partir du composé obtenu dans la Préparation 12. Huile jaune.

### Stade B : 5-(4-Cyano-butyl)-2,3-dihydro-1,4-benzoxathiine

Le composé obtenu au Stade A est réduit selon le procédé du Stade C de la Préparation 2. Huile incolore.

### Stade C : N-[5-(2,3-dihydro-1,4-benzoxathiin-5-yl)-pentyl]-acétamide

Le dérivé cyané saturé obtenu au Stade B (400 mg ; 1,71 mmol) est solubilisé dans le réacteur de l'appareil de Paar à l'aide d'anhydride acétique. L'acétate de sodium (211 mg ; 2,57 mmol ; 1,5 éq.) ainsi que le nickel de Raney (48 mg) sont alors introduits dans le milieu réactionnel. Le tout est chauffé à 50°C sous une pression initiale d'hydrogène de 40 psi durant 12 heures. Le solvant étant éliminé après retour à des conditions normales de température et de pression, l'extraction du produit est effectuée à l'acétate d'éthyle. Une purification par flash chromatographie CHCl₃/AcOEt (7:3) de la phase organique concentrée aboutit à l'amide du titre. Ce dernier précipitant à froid est lavé à l'éther diéthylique.
Point de fusion : 93°C

| Microanalyse Elémentaire : | C | H | N | S |
|---|---|---|---|---|
| % Calculé | 64,48 | 7,58 | 5,01 | 11,48 |
| % Trouvé | 64,62 | 7,67 | 5,09 | 11,19 |

### EXEMPLE 30 : N-[3-(2,3-Dihydro-1,4-benzoxathiin-5-yl)-propyl]-acétamide

### Stade A : 2-[3-(2,3-Dihydro-1,4-benzoxathiin-5-yl)-propyl]-isoindole-1,3-dione

On procède comme dans le Stade B de la Préparation 7 à partir du composé de la Préparation 9.
Point de fusion : 132°C

### Stade B : 3-(2,3-Dihydro-1,4-benzoxathiin-5-yl) propylamine

On procède comme dans le Stade C de la Préparation 7 à partir du composé obtenu au Stade A. Huile jaune.

### Stade C : N-[3-(2,3-Dihydro-1,4-benzoxathiin-5-yl)-propyl]-acétamide

On procède comme dans le Stade C de l'Exemple 1 à partir du composé obtenu au Stade B. Gomme jaune.

### EXEMPLE 31 : N-[3-(2,3-Dihydro-1,4-benzoxathlin-5-yl)propyl]-cyclopropane carboxamide

On procède comme dans l'Exemple 30 en remplaçant dans le Stade C l'anhydride acétique par l'anhydride cyclopropanecarboxylique.

### EXEMPLE 32 : N-[3-(2,3-Dihydro-1,4-benzoxathiin-5-yl)propyl]-N'-propylurée

On procède comme dans l'Exemple 23 à partir de l'amine obtenue au Stade B de l'Exemple 30.

### EXEMPLE 33 : N-[4-(2,3-Dihydro-1,4-benzoxathiin-5-yl)-butyl]-acétamide

### Stade A : 4-(2,3-Dihydro-1.4-benzoxathiin-5-yl)butylamine

On procède comme dans le Stade B de l'Exemple 3 à partir du composé obtenu dans la Préparation 10. Huile jaune.

### Stade B : N-[4-(2,3-Dihydro-1,4-benzoxathiin-5-yl)-butyl]-acétamide

On procède comme dans le Stade C de l'Exemple 1 à partir du composé obtenu dans le Stade A. Gomme jaune.

### EXEMPLE 34 : N-[2-(3,4-Dihydro-2H-1-benzothiopyran-8-yloxy)-éthyl]-acétamide

### Stade A : 2-[2-(2H-1-Benzothiopyran-8-yloxy)-éthyl]-isoindole-1,3-dione

On procède comme dans le Stade A de l'Exemple 1 à partir du composé de la Préparation 8.
Point de fusion : 154°C

### Stade B : 2-(3,4-Dihydro-2H-1-benzothiopyran-8-yloxy) éthylamine

On procède comme dans le Stade B de l'Exemple 1 à partir du composé obtenu au Stade A.
Point de fusion : 45°C

### Stade C : N-[2-(3,4-Dihydro-2H-1-benzothiopyran-8-yloxy)-éthyl]-acétamide

On procède comme dans le Stade C de l'Exemple 1 à partir du composé obtenu au Stade B.
Point de fusion : 117°C

### EXEMPLE 35 : N-Méthyl-[4-(2,3-dihydro-1,4-benzoxathiin-5-yl)]-butanamide

On procède comme dans le Stade B de l'Exemple 4 partir du composé de la Préparation 11.
Point de fusion : 94°C

Les Exemples 36, 37 et 38 sont obtenus de la même façon que l'Exemple 35 en condensant l'amine appropriée.

### EXEMPLE 36 : N-Allyl-4-(2,3-dihydro-1,4-benzoxathiin-5-yl)butanamide

### EXEMPLE 37 : N-Propyl-[4-(2,3-dihydro-1,4-benzoxathiin-5-yl)]-butanamide

Point de fusion : 93°C

| Microanalyse Elémentaire : | C | H | N | S |
|---|---|---|---|---|
| % Calculé | 64,48 | 7,58 | 5,01 | 11,48 |
| % Trouvé | 64,79 | 7,59 | 5,08 | 11,39 |

### EXEMPLE 38 : N-Ethyl-[4-(2,3-dihydro-1,4-benzoxathiin-5-yl)]-butanamide

Point de fusion : 86°C

| Microanalyse Elémentaire : | C | H | N | S |
|---|---|---|---|---|
| % Calculé | 63.37 | 7,22 | 5,28 | 12,08 |
| % Trouvé | 63,77 | 7,27 | 5,36 | 11,98 |

### EXEMPLE 39 : N-[2-(3,4-Dihydro-2H-1-benzopyran-8-yloxy)-éthyl]-acétamide

### Stade A : 1-Méthoxy-2-(2-propynyloxy)benzène

La condensation du bromure de propargyle (en solution à 10 % dans le toluène) (8,97 ml ; 100,7 mmol : 1 éq.) sur le gaïacol (10 g : 80,5 mmol) s'effectue sous atmosphère d'argon au sein de l'acétone (150 ml) en présence de carbonate de potassium (13,36 g ; 96,7 mmol ; 1,2 éq.). Lorsque 20 heures de reflux se sont écoulées, le solvant est éliminé sous pression réduite après filtration des sels sur célite. Lors de l'extraction du concentrat au dichlorométhane la phase organique est lavée successivement avec une solution de soude à 5 % puis à 10 %. Cette dernière séchée sur sulfate de magnésium puis évaporée est purifiée sur gel de silice à l'aide d'un mélange AcOEt/EP (3:7). L'acétylénique se présente alors sous la forme d'une huile jaune.

### Stade B : 8-Méthoxy-2H-1-benzopyrane

La cyclisation de l'acétylénique obtenu dans le Stade A (3 g ; 18,5 mmol) nécessite un chauffage avoisinant 240 °C durant 5 heures 30. Pour ce faire le choix d'un solvant tel que le triéthylène glycol (74 ml : 555 mmol ; 30 éq.) s'avère indispensable, de plus il est souhaitable d'opérer en milieu anhydre. La réaction étant refroidie à température ambiante, 50 ml d'éther diéthylique sont introduits pour extraire le produit. Après 4 lavages successifs de la phase organique avec une solution saturée au chlorure de sodium, le concentrat est purifié sur colonne de silice. Une élution à l'éther de pétrole puis par un mélange AcOEt/EP (1:9) fournit le chromène sous l'aspect d'une huile jaune.

### Stade C : 8-Méthoxy-3,4-dihydro-2H-1-benzopyrane

Le composé obtenu au Stade B (1 g : 6,84 mmol) en solution dans l'acide acétique glacial (10 ml) est versé dans le réacteur de l'appareil de Paar. Le palladium sur charbon à 10% (10% en masse ; 100 mg) faisant office de catalyseur est additionné : le tout est alors soumis à une pression d'hydrogène de 45 psi durant 12 heures à température ambiante. Après filtration, le solvant est éliminé sous pression réduite puis on procède à une extraction au dichlorométhane. La phase organique récupérée, séchée sur sulfate de magnésium est déposée après concentration sur une colonne de silice éluée au dichlorométhane pur. Le produit du titre est obtenu pur sous forme d'une huile de couleur jaune.

### Stade D : 8-Hydroxy-3,4-dihydro-2H-1-benzopyrane

Le composé obtenu au Stade C (1 g ; 6,09 mmol) étant en solution dans l'acide acétique glacial (10.2 ml). l'acide bromhydrique (en solution à 48% dans l'eau) (3,7 éq. ; 47 mmol ; 2,54 ml) est ajouté goutte à goutte. Le phénol est totalement déprotégé après 5 heures de reflux. Après élimination du solvant sous pression réduite, le résidu sec est repris en milieu aqueux puis neutralisé par une solution saturée d'hydrogénocarbonate de sodium jusqu'à un pH de 8. Une extraction à l'acétate d'éthyle fournit après séchage et concentration une huile orange. Cette dernière purifiée par une colonne de silice AcOEt/EP (3:7) est de couleur jaune.

### Stade E : 8-(2-Bromoéthoxy)-3,4-dihydro-2H-1-benzopyrane

On procède de la même façon que dans le Stade A de la Préparation 7 à partir du composé obtenu au Stade D.
Point de fusion : 70°C

### Stade F : 2-[2-(2H-1-Benzopyran-8-yloxy)-éthyl]-isoindole-1,3-dione

On procède comme dans le Stade A de l'Exemple 1 à partir du composé obtenu au Stade E.
Point de fusion : 104°C

### Stade G : 2-(3,4-Dihydro-2H-1-benzopyran-8-yloxy) éthylamine

On procède comme dans le Stade B de l'Exemple 1 à partir du composé obtenu au Stade F.

### Stade H : N-[2-(3,4-Dihydro-2H-1-benzopyran-8-yloxy)-éthyl]-acétamide

On procède comme dans le Stade C de l'Exemple 1 à partir du composé obtenu au Stade G.
Point de fusion : 111°C

### EXEMPLE 40 : N-Méthyl-4-(1,4-benzodioxin-5-yl)butanamide

### Stade A : Ethyl-3-(1,4-benzodioxin-5-yl)-2-propenoate

4,13 g (12 mmol) de carbéthoxyméthylènetriphénylphosphorane sont ajoutés à une solution de 1.3 g (8 mmol) de l'aldéhyde obtenu au Stade E de la Préparation 5 dans 20 ml de toluène puis le milieu est agité sous argon et à température ambiante pendant 2 heures. Le solvant est évaporé sous vide puis le résidu obtenu est purifié sur colonne de silice (éluant: AcOEt/EP : 85/15) pour aboutir à l'ester du titre de configuration E sous forme d'un solide jaune.
Point de fusion : 58°C

### Stade B : Ethyl-3-(1.4-benzodioxin-5-yl)-2-propanoate

4.2 g (172 mmol) de magnésium sont ajoutés à une solution de 1 g (4,3 mmol) de l'ester obtenu au Stade A dans 30 ml de méthanol anhydre. Après refroidissement du milieu, le mélange réactionnel est agité pendant 24 heures puis les sels sont dissous à l'aide d'une solution d'acide chlorhydrique 6 N. La phase aqueuse est extraite à l'aide de dichlorométhane puis la phase organique est séchée sur sulfate de magnésium. Après évaporation du solvant sous vide, le résidu obtenu est purifié sur colonne de silice (éluant : AcOEt/EP : 10/90) pour conduire à l'ester du titre sous forme d'une huile claire.

### Stade C : 3-(1,4-benzodioxin-5-yl)-1-propanol

0.233 g (6,14 mmol) d'hydrure de lithium et d'aluminium sont ajoutés à une solution de 0,9 g (4,09 mmol) de l'ester obtenu au Stade B dans 30 ml d'éther anhydre puis le milieu est porté à reflux pendant 30 minutes. Après refroidissement, la solution est hydrolysée selon la procédure habituelle puis les sels sont filtrés. Le filtrat est ensuite concentré sous vide puis le produit brut est purifié par chromatographie sur colonne de silice (éluant : AcOEt/EP : 40:60) pour conduire à l'alcool du titre sous forme d'une huile claire.

### Stade D : 3-(1.4-benzodioxin-5-yl)propyl-4-méthyl-1-benzènesulfonate

1,28 g (6,72 mmol) de chlorure de tosyle sont ajoutés à une solution de 0,86 g (4,448 mmol) de l'alcool obtenu au Stade C et de 1,9 ml (13,44 mmol) de triéthylamine dans 20 ml de dichlorométhane anhydre. Le milieu est agité pendant 24 heures à température ambiante puis le solvant est évaporé sous vide. Le produit brut est purifié sur colonne de silice (éluant : AcOEt/EP : 5/95 puis 10/90) pour aboutir au tosylate du titre sous forme d'un sirop clair.

### Stade E : 4-(1.4-benzodioxin-5-yl)butanenitrile

Une solution de 1,065g (3,07mol) du tosylate obtenu au stade D ainsi que 0,5 g (7,7 mmol) de cyanure de potassium est chauffée à reflux au sein de 15 ml de DMF, sous argon, pendant 4 heures. Le milieu est laissé refroidir puis le solvant est évaporé sous vide. Le résidu obtenu est repris dans de l'eau puis la phase aqueuse est extraite à l'aide de dichlorométhane. Après séchage sur sulfate de magnésium, la phase organique est concentrée sous pression réduite. Une chromatographie sur gel de silice (éluant : EP/AcOEt : 90/10) permet d'obtenir le nitrile du titre sous forme d'une huile claire.

### Stade F : Acide 4-(1,4-benzodioxin-5-yl)butanoïque

0.58 g (2,90 mmol) du nitrile obtenu au stade E dans 20 ml d'une solution d'hydroxyde de sodium à 10.% est chauffé à reflux pendant 20 heures. Après évaporation du solvant sous vide, les sels sont dissous dans 20 ml d'une solution d'acide chlorhydrique 1 N. La phase aqueuse est extraite à l'aide d'acétate d'éthyle puis la phase organique est séchée sur sulfate de magnésium. L'évaporation du solvant sous pression réduite conduit à l'acide du titre sous forme d'un solide blanc avec un rendement quantitatif.
Point de fusion : 75-76 °C.

### Stade G : N-méthyl-4-(1,4-benzodioxin-5-yl)butanamide

0.1 *g (0,75* mmol) d'hydroxybenzotriazole (HOBt) et 0,14 g (0,75 mmol) de chlorhydrate du 1-(3-diméthylaminopropyl)-3-éthylcarbodiimide (EDCI) sont introduits à 0 °C dans un milieu réactionnel comprenant 0,15 g (0,68 mmol) de l'acide obtenu au stade F en solution dans 3 ml de DMF anhydre. Après addition de 1,02 mmol de méthylamine en solution à 10 % dans le benzène, l'agitation est maintenue pendant 24 heures puis le solvant est évaporé sous vide. Le résidu obtenu est repris dans du dichlorométhane puis lavé à l'eau. La phase organique est séchée sur sulfate de magnésium puis concentrée sous vide. Une chromatographie sur gel de silice (éluant : AcOEt/EP : 70/30) permet d'isoler l'amide du titre sous forme d'une huile claire.

### EXEMPLE 41 : N-Propyl-4-(1,4-benzodioxin-5-yl)butanamide

Une suspension de 0.29 g (2,6 mmol) de chlorhydrate de n-propylamine dans 7 ml de N.N-diméthylformamide anhydre est refroidie à 0 °C puis 0,33 g (3,54 mmol) de N.N-diméthylaminopyridine sont ajoutés au milieu. Après addition de 0,41 g (2,36 mmol) de l'acide obtenu dans le stade F de l'Exemple 40. 0,5 g (2,6 mmol) d'EDCI est ajouté au milieu. La solution est agitée pendant 12 heures sous argon et à température ambiante puis le solvant est évaporé sous vide. Le résidu est repris dans un mélange eau/acétate d'éthyle (1/1) puis la phase aqueuse est extraite à l'acétate d'éthyle. La phase organique est séchée sur sulfate de magnésium puis concentrée sous pression réduite. Une chromatographie sur gel de silice (éluant : AcOEt/EP : 70/30) permet de conduire à l'amide du titre sous forme d'un sirop clair.

### EXEMPLE 42 : N-Propyl-4-(2,3-dihydro-1,4-benzodioxin-5-yl)butanamide

Le chlorhydrate de propylamine (134 mg, 1.5 éq) est dissous dans 5 ml de DMF et placé dans la glace. L'acide obtenu dans le stade A de l'Exemple 11 (260 mg) en solution dans 1 ml de DMF est alors ajouté au milieu réactionnel. Puis l'EDCI (247 mg, 1,1 éq) est additionné. L'agitation est maintenue toute la nuit en laissant remonter la température.
La DMF est évaporée, puis le résidu est hydrolysé et extrait à l'acétate d'éthyle. La phase organique est séchée sur MgSO₄.
Le produit, purifié par flash chromatographie (éluant : EP/AcOEt, 8/2), est obtenu sous forme d'un solide blanc.

Point de fusion : 57-58°C

| Microanalyse Elémentaire : | C | H | N |
|---|---|---|---|
| % Calculé | 68,41 | 8,04 | 5,32 |
| % Trouvé | 68,59 | 8,14 | 5,38 |

### EXEMPLE 43 : N-[3-(2,3-Dihydro-1,4-benzodioxin-5-yl)propyl]butanamide

L'amine précédemment obtenue au cours du stade B de l'Exemple 8 (300 mg) est dissoute dans 7 ml de CH₂Cl₂. A 0°C, la triéthylamine (0.708 ml, 1.2 éq) et le chlorure de butanoyle (0,248 ml, 1.2 éq) sont ajoutés. Le mélange réactionnel est agité à 0°C pendant 5 heures. Le mélange est ensuite acidifié avec de l'acide chlorhydrique 1 N, puis extrait au CH₂Cl₂. La phase organique est lavée avec une solution d'hydrogénocarbonate de sodium saturée, puis séchée sur MgSO₄.
Le produit purifié par flash chromatographie (éluant : EP/AcOEt, 8/2), est obtenu sous forme d'huile incolore.

### EXEMPLE 44 : N-Méthyl-3-(2,3-dihydro-1,4-benzoxathiin-5-yl)propanamide

### Stade A : Acide 3-(2,3-dihydro-1,4-benzoxathiin-5-yl)propanoïque

L'aldéhyde obtenu dans la Préparation 12 est soumis à une oxydation au nitrate d'argent en milieu basique (G. Guillaumet et al., Can. J. Chem., 1992, 70, 828-835) pour conduire au produit du titre.

### Stade B : N-Méthyl-3-(2,3-dihydro-1,4-benzoxathiin-5-yl)propanamide

On procède comme dans l'Exemple 42 en remplaçant le chlorhydrate de propylamine par le chlorhydrate de méthylamine.
Point de fusion : 98-99°C

| Microanalyse Elémentaire : | C | H | N | S |
|---|---|---|---|---|
| % Calculé | 60,73 | 6,37 | 5,90 | 13,51 |
| % Trouvé | 60,46 | 6,35 | 5,75 | 13,53 |

### EXEMPLE 45 : N-Méthyl-5-(2,3-dihydro-1,4-benzoxathiin-5-yl)pentanamide

On procède comme dans les stades B, C. D et E de l'Exemple 13, à partir du composé obtenu dans la Préparation 12.
Point de fusion : 66°C

| Microanalyse Elémentaire : | C | H | N | S |
|---|---|---|---|---|
| % Calculé | 63,36 | 7,22 | 5,28 | 12,08 |
| % Trouvé | 63,65 | 71,9 | 5,35 | 12,40 |

### ETUDE PHARMACOLOGIQUE

### EXEMPLE A : Etude de la toxicité aiguë

La toxicité aiguë a été appréciée après administration orale à des lots de 8 souris (26 ± 2 grammes). Les animaux ont été observés à intervalles réguliers au cours de la première journée et quotidiennement pendant les deux semaines suivant le traitement. La DL 50. entraînant la mort de 50 % des animaux, a été évaluée et a montré la faible toxicité de la plupart des composés de l'invention.

### EXEMPLE B : Etude de liaison aux récepteurs de la mélatonine

### 1) Etude sur des cellules de la pars tuberalis de mouton

Les études de liaison aux récepteurs de la mélatonine des composés de l'invention ont été réalisées selon les techniques classiques sur les cellules de la pars tuberalis de mouton. La pars tuberalis de l'adénohypophyse est en effet caractérisée, chez les mammifères, par une haute densité en récepteurs de la mélatonine (Journal of Neuroendocrinology, 1, pp 1-4, 1989).

### Protocole

1) Les membranes de pars tuberalis de mouton sont préparées et utilisées comme tissu cible dans des expériences de saturation pour déterminer les capacités et affinités de liaison pour la [¹²⁵I] - iodomélatonine.
2) Les membranes de pars tuberalis de mouton sont utilisées comme tissu cible, avec les différents composés à tester, dans des expériences de liaison compétitive par rapport à la mélatonine.

Chaque expérience est réalisée en triple et une gamme de concentrations différentes est testée pour chaque composé. Les résultats permettent de déterminer, après traitement statistique, les affinités de liaison du composé testé.

### Résultats

Il apparaît que les composés de l'invention possèdent une puissante affinité pour les récepteurs de la mélatonine.

### 2) Etude sur des membranes d'homogénat de cerveau de poulet (Gallus Domesticus)

Les animaux utilisés sont des poulets (Gallus domesticus) âgés de 12 jours. Ils sont sacrifiés entre 13 et 17 heures le jour de leur arrivée. Les cerveaux sont rapidement prélevés et congelés à - 200°C puis conservés à - 80°C. Les membranes sont préparées selon la méthode décrite par Yuan et Pang (Journal of Endocrinology, 128, pp 475-482. 1991). La [¹²⁵I] mélatonine est incubée en présence des membranes dans une solution tamponnée à pH 7,4 pendant 60 min à 25°C. A l'issue de cette période, la suspension membranaire est filtrée (Whatman GF/C). La radioactivité retenue sur le filtre est déterminée à l'aide d'un compteur à scintillation liquide Beckman® LS 6000.

Les produits utilisés sont :
- 2-[¹²⁵I] mélatonine
- mélatonine
- produits courants
- molécules originales

En screening primaire, les molécules sont testées à 2 concentrations (10⁻⁷ et 10⁻⁵ M). Chaque résultat est la moyenne de 3 mesures indépendantes. Les molécules actives retenues d'après les résultats du screening primaire ont fait l'objet d'une détermination quantitative de leur efficacité (IC₅₀). Elles sont utilisées à 10 concentrations différentes. Ainsi les valeurs d'IC₅₀ trouvées pour les composés préférés de l'invention, qui correspondent aux valeurs de l'affinité montrent que la liaison des composés testés est très puissante.

### EXEMPLE C : Test des quatre plaques

Les produits de l'invention sont administrés par voie oesophagienne à des lots de dix souris. Un lot reçoit du sirop de gomme. Trente minutes après l'administration des produits à étudier, les animaux sont placés dans des habitacles dont le plancher comprend quatre plaques métalliques. Chaque fois que l'animal passe d'une plaque à l'autre, il reçoit une légère décharge électrique (0,35 mA). Le nombre de passages est enregistré pendant une minute.

Après administration, les composés de l'invention augmentent de façon significative le nombre de passages ce qui montre l'activité anxiolytique des dérivés de l'invention.

### EXEMPLE D : Action des composés de l'invention sur les rythmes circadiens d'activité locomotrice du rat

L'implication de la mélatonine dans l'entraînement, par l'alternance jour/nuit, de la plupart des rythmes circadiens physiologiques, biochimiques et comportementaux a permis d'établir un modèle pharmacologique pour la recherche de ligands mélatoninergiques.

Les effets des molécules sont testés sur de nombreux paramètres et, en particulier, sur les rythmes circadiens d'activité locomotrice qui représentent un marqueur fiable de l'activité de l'horloge circadienne endogène.

Dans cette étude, on évalue les effets de telles molécules sur un modèle expérimental particulier, à savoir le rat placé en isolement temporel (obscurité permanente).

### Protocole expérimental

Des rats mâles Long Evans âgés de un mois sont soumis dès leur arrivée au laboratoire à un cycle lumineux de 12h de lumière par 24h (LD 12 : 12).
Après 2 à 3 semaines d'adaptation, ils sont placés dans des cages équipées d'une roue reliée à un système d'enregistrement afin de détecter les phases d'activité locomotrice et de suivre ainsi les rythmes nycthéméraux (LD) ou circadiens (DD).
Dès que les rythmes enregistrés témoignent d'un entraînement stable par le cycle lumineux LD 12 : 12, les rats sont mis en obscurité permanente (DD).
Deux à trois semaines plus tard, lorsque le libre-cours (rythme reflétant celui de l'horloge endogène) est clairement établi, les rats reçoivent une administration quotidienne de la molécule à tester.

Les observations sont réalisées grâce à la visualisation des rythmes d'activité :
- entraînement des rythmes d'activité par le rythme lumineux.
- disparition de l'entraînement des rythmes en obscurité permanente,
- entraînement par l'administration quotidienne de la molécule ; effet transitoire ou durable.

Un logiciel permet :
- de mesurer la durée et l'intensité de l'activité, la période du rythme chez les animaux en libre cours et pendant le traitement,
- de mettre éventuellement en évidence par analyse spectrale l'existence de composants circadiens et non circadiens (ultradiens par exemple).

### Résultats :

Il apparaît clairement que les composés de l'invention permettent d'agir de façon puissante sur le rythme circadien *via* le système mélatoninergique.

### EXEMPLE E : Activité Antiarythmique

### Protocole

(Ref: LAWSON J.W. et al. J. Pharmacol. Expert. Therap., 1968, 160, pp 22-31)

La substance testée est administrée en intrapéritonéal à un groupe de 3 souris 30 min avant l'exposition à une anesthésie par le chloroforme. Les animaux sont ensuite observés pendant 15 min. L'absence d'enregistrement d'arythmies et de fréquences cardiaques supérieures à 200 battements / min (témoin : 400-480 battements / min) chez deux animaux au moins indique une protection significative.

### EXEMPLE F : Composition pharmaceutique : Comprimés

| | |
|---|---|
| 1000 comprimés dosés à 5 mg de N-Méthyl-[4-(2,3-dihydro-1,4-benzoxathiin-5-yl)]-butanamide (Exemple 35) | 5 g |
| Amidon de blé | 20 g |
| Amidon de maïs | 20 g |
| Lactose | 30 g |
| Stéarate de magnésium | 2 g |
| Silice | 1 g |
| Hydroxypropylcellulose | 2 g |

## Revendications

1. Composés de formule (I) : 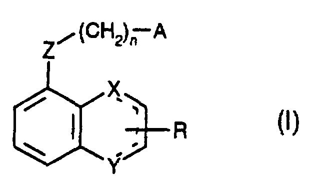 dans laquelle :
◆ X et Y, identiques ou différents, représentent un atome de soufre, un atome d'oxygène, un groupement CHq (où q vaut 0, 1 ou 2), SO ou SO₂, étant entendu que X et Y ne peuvent représenter simultanément un groupement CHq (où q vaut 0, 1 ou 2),
◆ Z représente un atome d'oxygène ou un groupement CH₂,
◆ n est égal à 0, 0, 1, 2, 3 ou 4,
◆ A représente
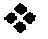 un groupement 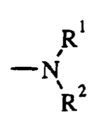 dans lequel
R¹ représente un atome d'hydrogène ou un groupement alkyle (C₁-C₆) linéaire ou ramifié.
et R² représente un groupement 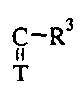 dans lequel T représente un atome de soufre ou un atome d'oxygène et R³ représente
- un groupement R'³ représentant un atome d'hydrogène, un groupement alkyle (C₁-C₆) linéaire ou ramifié éventuellement substitué, un groupement alcényle (C₂-C₆) linéaire ou ramifié éventuellement substitué, un groupement alcynyle (C₂-C₆) linéaire ou ramifié éventuellement substitué, un groupement cycloalkyle (C₃-C₈), cycloalkyle (C₃-C₈) substitué, cycloalkylalkyle, cycloalkylalkyle substitué, aryle, arylalkyle,
- ou un groupement 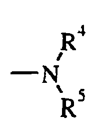
dans lequel R⁴ représente un atome d'hydrogène ou un groupement alkyle (C₁-C₆) linéaire ou ramifié et R⁵ représente un atome d'hydrogène, un groupement R'⁵ représentant un groupement alkyle (C₁-C₆) linéaire ou ramifié éventuellement substitué, un groupement alcényle (C₂-C₆) linéaire ou ramifié éventuellement substitué, un groupement alcynyle (C₂-C₆) linéaire ou ramifié éventuellement substitué, un groupement cycloalkyle (C₃-C₈). cycloalkyle (C₃-C₈) substitué, cycloalkylalkyle, cycloalkylalkyle substitué, aryle, ou arylalkyle,
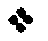 ou un groupement 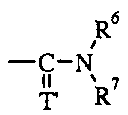 dans lequel
T' représente un atome de soufre ou un atome d'oxygène
R⁶ représente un atome d'hydrogène ou un groupement alkyle (C₁-C₆) linéaire ou ramifié
et R⁷ représente un atome d'hydrogène, un groupement alkyle (C₁-C₆) linéaire ou ramifié éventuellement substitué, un groupement alcényle (C₂-C₆) linéaire ou ramifié éventuellement substitué, un groupement alcynyle (C₂-C₆) linéaire ou ramifié éventuellement substitué, un groupement cycloalkyle (C₃-C₈), cycloalkyle (C₃-C₈) substitué, cycloalkylalkyle, cycloalkylalkyle substitué, aryle, ou arylalkyle.
◆ R représente un atome d'hydrogène, un groupement alkyle (C₁-C₆) linéaire ou ramifié éventuellement substitué, un groupement aryle ou arylalkyle,
◆ la représentation 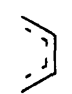 signifie que ces liaisons peuvent être simples ou doubles, étant entendu que deux liaisons adjacentes ne peuvent être doubles simultanément et que la valence des atomes est respectée,
étant entendu que :
- le terme "aryle" désigne un groupement phényle ou naphtyle éventuellement substitué par un ou plusieurs atomes d'halogène, groupements OH, alkyle (C₁-C₆) linéaire ou ramifié, alkoxy (C₁-C₆) linéaire ou ramifié, cyano, nitro, amino ou trihalogénoalkyle,
- le terme "arylalkyle" désigne un groupement alkyle (C₁-C₆) linéaire ou ramifié substitué par un groupement aryle tel que défini ci-dessus,
- le terme "cycloalkylalkyle" désigne un alkyle (C₁-C₆) linéaire ou ramifié substitué par un ou plusieurs groupements cycloalkyle (C₃-C₈),
- le terme substitué affecté aux termes "alkyle", "alcényle" et "alcynyle" signifie que ce groupement est substitué par un ou plusieurs atomes d'halogène, groupements OH, ou alkoxy,
- le terme substitué affecté aux termes "cycloalkyle" et "cycloalkylalkyle" signifie que la partie cyclique est substituée par un ou plusieurs atomes d'halogène, groupements alkyle (C₁-C₆) linéaire ou ramifié, alkoxy (C₁-C₆) linéaire ou ramifié, phényle, hydroxy ou oxo,
étant entendu que
. lorsque X et Z représentent simultanément un groupement CH₂, R représente un atome d'hydrogène et A représente un groupement NR'R². alors Y ne peut représenter un atome d'oxygène,
. lorsque Z représente un atome d'oxygène. alors n est différent de zéro,
. lorsque Z représente un atome d'oxygène et n vaut un, alors A ne peut représenter un groupement CONEt₂,
. lorsque Z représente un groupement CH₂, n vaut 1 et A représente un groupement -NR¹CSNR⁴R⁵, alors R⁵ ne peut représenter un groupement aryle,
. lorsque X et Y représentent simultanément un atome d'oxygène, que les liaisons en pointillés sont saturées. R représente un atome d'hydrogène ou un groupement CH₂OH, alors A est différent d'un groupement NR¹R^{2a} dans lequel R¹ est tel que défini précédemment et R^{2a} représente un groupement benzoyle éventuellement substitué.
leurs énantiomères et diastéréoisomères, ainsi que leurs sels d'addition à un acide ou une base pharmaceutiquement acceptable.

2. Composés de formule (I) selon la revendication 1 dans laquelle R représente un atome d'hydrogène, leurs énantiomères et diastéréoisomères, ainsi que leurs sels d'addition à un acide ou une base pharmaceutiquement acceptable.

3. Composés de formule (I) selon la revendication 1 dans laquelle X et Y représentent simultanément un atome d'oxygène, leurs énantiomères et diastéréoisomères, ainsi que leurs sels d'addition à un acide ou une base pharmaceutiquement acceptable.

4. Composés de formule (I) selon la revendication 1 dans laquelle X représente un atome de soufre et Y représente un atome d'oxygène, leurs énantiomères et diastéréoisomères, ainsi que leurs sels d'addition à un acide ou une base pharmaceutiquement acceptable.

5. Composés de formule (I) selon la revendication 1 dans laquelle X représente un atome de soufre ou d'oxygène et Y représente un groupement CH_{q} (où q vaut 0, 1 ou 2), leurs énantiomères et diastéréoisomères, ainsi que leurs sels d'addition à un acide ou une base pharmaceutiquement acceptable.

6. Composés de formule (I) selon la revendication 1 dans laquelle Z représente un atome d'oxygène, leurs énantiomères et diastéréoisomères, ainsi que leurs sels d'addition à un acide ou une base pharmaceutiquement acceptable.

7. Composés de formule (I) selon la revendication 1 dans laquelle Z représente un groupement CH₂, leurs énantiomères et diastéréoisomères, ainsi que leurs sels d'addition à un acide ou une base pharmaceutiquement acceptable.

8. Composés de formule (I) selon la revendication 1 dans laquelle A représente un groupement NR¹COR³, leurs énantiomères et diastéréoisomères, ainsi que leurs sels d'addition à un acide ou une base pharmaceutiquement acceptable.

9. Composés de formule (I) selon la revendication 1 dans laquelle A représente un groupement CONR⁶R⁷. leurs énantiomères et diastéréoisomères, ainsi que leurs sels d'addition à un acide ou une base pharmaceutiquement acceptable.

10. Composé de formule (I) selon la revendication 1 qui est le N-Méthyl-[4-(2.3-dihydro-1,4-benzoxathiin-5-yl)]-butanamide, ses énantiomères et diastéréoisomères, ainsi que ses sels d'addition à un acide ou une base pharmaceutiquement acceptable.

11. Composé de formule (I) selon la revendication 1 qui est le N-Ethyl-[4-(2,3-dihydro-1.4-benzoxathiin-5-yl)]-butanamide. ses énantiomères et diastéréoisomères, ainsi que ses sels d'addition à un acide ou une base pharmaceutiquement acceptable.

12. Composé de formule (I) selon la revendication 1 qui est le N-[3-(1,4-Benzodioxin-5-yl)-propyl]-n-butanamide. ses énantiomères et diastéréoisomères, ainsi que ses sels d'addition à un acide ou une base pharmaceutiquement acceptable.

13. Composé de formule (I) selon la revendication 1 qui est le N-Méthyl-[4-(2,3-dihydro-1,4-benzodioxin-5yl)]-butanamide. ses énantiomères et diastéréoisomères, ainsi que ses sels d'addition à un acide ou une base pharmaceutiquement acceptable.

14. Procédé de préparation des composés de formule (I) selon la revendication 1 **caractérisé en ce que** l'on prend comme produit de départ un composé de formule (II) : 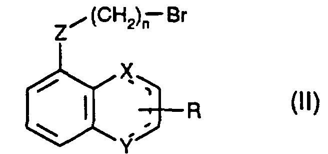 dans laquelle X, Y. Z. R et n sont tels que définis précédemment,
◆ que l'on soumet successivement à l'action d'un phtalimidure puis à une hydrazinolyse pour conduire au composé de formule (III) : 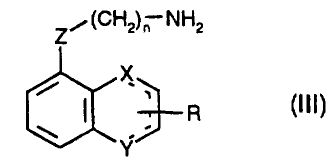 dans laquelle X, Y. Z, n et R sont tels que définis précédemment.
composé (III) pouvant être par ailleurs obtenu à partir du composé (II) par action d'un sel de cyanure ou un azoture suivi d'une réduction.
sur lequel on condense :
- un chlorure d'acyle de formule (IV) 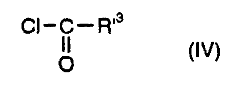 dans laquelle R'³ est tel que défini précédemment, ou l'anhydride d'acide (mixte ou symétrique) correspondant,
pour obtenir le composé de formule (I/a), cas particulier des composés de formule (I) : 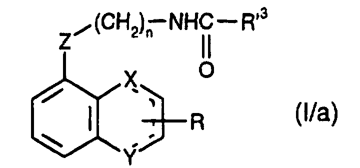 dans laquelle X. Y, Z, n, R et R'³ sont tels que définis précédemment,
qui peut être soumis à un agent de thionation pour obtenir le composé de formule (I/b), cas particuliers des composés de formule (I) : 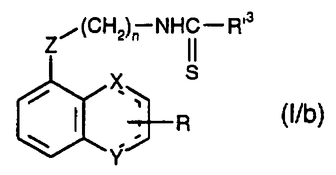 dans laquelle X, Y, Z, n, R et R'³ sont tels que définis précédemment,
- ou un composé de formule (V)
T=C=N-R⁵ (V)
dans laquelle T et R⁵ sont définis comme précédemment,
afin d'obtenir le composé de formule (I/c), cas particulier des composés de formule (I) : 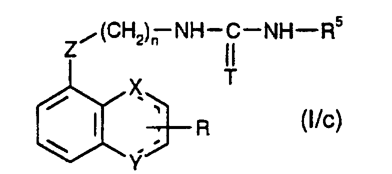 dans laquelle X, Y, Z, n, R, T et R⁵ sont définis comme précédemment,
l'ensemble des composés (I/a), (I/b) et (I/c) formant le composé de formule (I/d), cas particulier des composés de formule (I) : 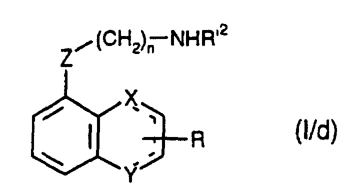 dans laquelle X, Y, Z, n et R sont tels que définis précédemment et R'² représente un groupement 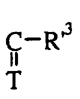 où T et R'³ sont définis comme précédemment ou un groupement 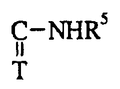 où T et R⁵ sont définis comme précédemment. composé (I/d) que l'on peut alkyler selon une technique classique d'alkylation grâce à un composé de formule (VI) :
Alk-W (VI)
dans laquelle Alk représente un groupement alkyle (C₁-C₆) linéaire ou ramifié et W représente un groupe partant comme un atome d'halogène ou un groupement tosyle,
ou grâce à un dialkylsulfate,
pour conduire au composé de formule (I/e), cas particulier des composés de formule (I) : 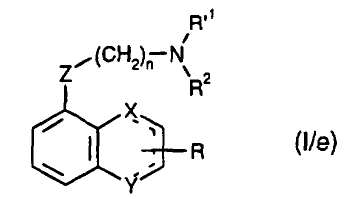 dans laquelle X, Y, Z, n, R et R² sont tels que définis précédemment, et R'¹ représente un groupement alkyle (C₁-C₆) linéaire ou ramifié,
◆ ou que l'on soumet successivement à l'action d'un sel d'acide carboxylique puis à une saponification pour conduire au composé de formule (VII) : 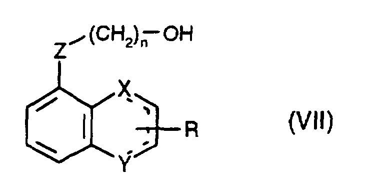 dans laquelle X, Y, Z, n et R sont tels que définis précédemment,
qui est :
- soit oxydé en aldéhyde correspondant pour obtenir le composé de formule (VIII) : 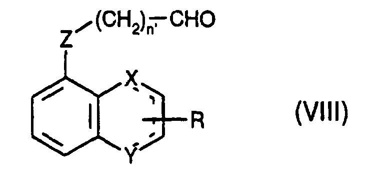 dans laquelle X, Y. Z, et R sont définis comme précédemment, et n' vaut 0, 1, 2 ou 3,
qui est oxydé en acide correspondant puis soumis à l'action d'une amine H₂NR⁷ où R⁷ est tel que défini précédemment,
pour conduire au composé de formule (I/f), cas particulier des composés de formule (I): 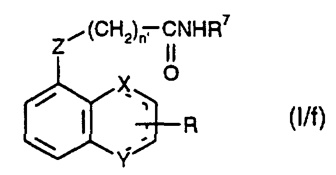 dans laquelle X, Y, Z, n', R et R⁷ sont tels que définis précédemment,
qui peut être soumis à un agent de thionation pour obtenir le composé (I/g), cas particulier des composés de formule (I) : 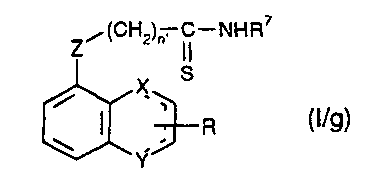 dans laquelle X. Y. Z, n', R et R⁷ sont tels que définis précédemment,
l'ensemble des composés (I/f) et (I/g) formant le composé de formule (I/h) : 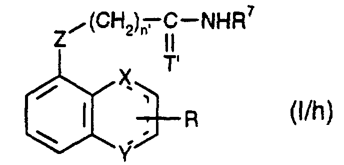 dans laquelle X, Y, Z, T', n', R et R⁷ sont tels que définis précédemment, composé (I/h) qui peut être alkylé selon une technique classique d'alkylation pour conduire au composé de formule (I/i), cas particulier des composés de formule (I) : 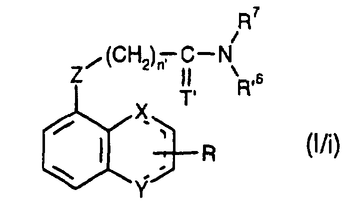 dans laquelle X, Y, Z, T', n', R et R⁷ sont tels que définis précédemment, et R'⁶ représente un groupement alkyle (C₁-C₆) linéaire ou ramifié,
- soit transformé en groupe partant comme un tosylate puis substitué
. par un cyanure pour conduire au composé de formule (IX): 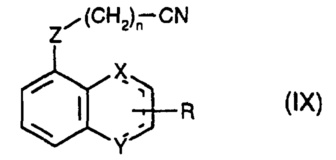 dans laquelle X. Y. Z, R et n sont tels que définis précédemment,
qui est successivement soumis à une hydrolyse et à la condensation d'une amine H₂NR⁷ pour conduire au composé de formule (I/j) cas particulier des composés de formule (I) : 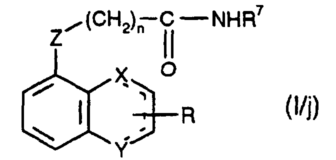 dans laquelle X, Y. Z, n, R et R⁷ sont tels que définis précédemment.
qui peut être soumis à un agent de thionation pour obtenir le composé (I/k), cas particulier des composés de formule (I) : 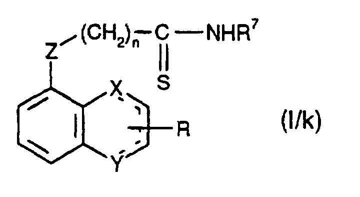 dans laquelle X, Y, Z, n, R et R⁷ sont tels que définis précédemment,
l'ensemble des composés (I/j) et (I/k) formant le composé de formule (I/l), cas particulier des composés de formule (I): 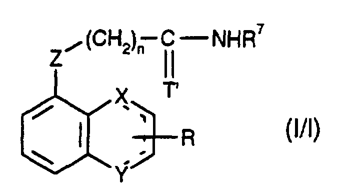 dans laquelle X. Y, Z, n. T', R et R⁷ sont tels que définis précédemment,
qui peut être alkylé selon une technique classique d'alkylation pour conduire au composé de formule (I/m), cas particulier des composés de formule (I) : 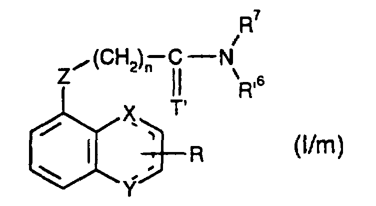 dans laquelle X, Y, Z, n, T', R et R⁷ sont tels que définis précédemment, et R'⁶ représente un groupement alkyle (C₁-C₆) linéaire ou ramifié,
. ou par un azoture suivi d'une réduction pour conduire au composé de formule (III).
l'ensemble des composés (I/a) à (I/m) formant les composés de formule générale (I) qui peuvent être purifiés selon une technique classique de purification, dont on sépare, le cas échéant, les isomères selon une technique classique de séparation, que l'on transforme, si on le souhaite en leurs sels d'addition à un acide ou à une base pharmaceutiquement acceptable.

15. Compositions pharmaceutiques contenant les produits de formule (I) selon l'une quelconque des revendications 1 à 13 ou un de leurs sels d'addition avec un acide ou une base pharmaceutiquement acceptable en combinaison avec un ou plusieurs excipients pharmaceutiquement acceptables.

16. Compositions pharmaceutiques selon la revendication 15 utiles pour le traitement des troubles liés au système mélatoninergique.

## Patentansprüche

1. Verbindungen der Formel (I): 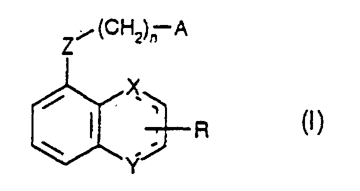 in der:
◆ X und Y, die gleichartig oder verschieden sind, ein Schwefelatom, ein Sauerstoffatom, eine Gruppe CH_{q} (worin q den Wert 0, 1 oder 2 besitzt), SO oder SO₂ bedeutet, mit der Maßgabe, daß X und Y nicht gleichzeitig eine Gruppe CH_{q} (worin q 0, 1 oder 2 darstellt) bedeuten,
◆ Z ein Sauerstoffatom oder eine CH₂-Gruppe bedeutet,
◆ n 0, 1, 2, 3 oder 4 darstellt,
◆ A
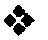 eine Gruppe 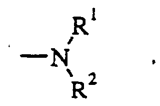 in der
R¹ ein Wasserstoffatom oder eine geradkettige oder verzweigte (C₁-C₆)-Alkylgruppe darstellt,
und R² eine Gruppe 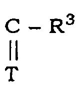 darstellt, in der T ein Schwefelatom oder ein Sauerstoffatom bedeutet und R³
- eine Gruppe R'³, die ein Wasserstoffatom, eine geradkettige oder verzweigte gegebenenfalls substituierte (C₁-C₆)-Alkylgruppe, eine geradkettige oder verzweigte gegebenenfalls substituierte (C₂-C₆)-Alkenylgruppe, eine geradkettige oder verzweigte gegebenenfalls substituierte (C₂-C₆)-Alkinylgruppe, eine (C₃-C₈)-Cycloalkylgruppe, eine substituierte (C₃-C₈)-Cycloalkylgruppe, eine Cycloalkylalkylgruppe, eine substituierte Cycloalkylalkylgruppe, eine Arylgruppe oder eine Arylalkylgruppe darstellt,
- oder eine Gruppe 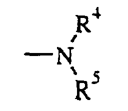 bedeutet, worin R⁴ ein Wasserstoffatom oder eine geradkettige oder verzweigte (C₁-C₆)-Alkylgruppe und R⁵ ein Wasserstoffatom, eine Gruppe R'⁵, die eine geradkettige oder verzweigte gegebenenfalls substituierte (C₁-C₆)-Alkylgruppe, eine geradkettige oder verzweigte gegebenenfalls substituierte (C₂₋C₆)-Alkenylgruppe, eine geradkettige oder verzweigte gegebenenfalls substituierte (C₂-C₆)-Alkinylgruppe, eine (C₃-C₈)-Cycloalkylgruppe, eine substituierte (C₃-C₈)-Cycloalkylgruppe, eine Cycloalkylalkylgruppe, eine substituierte Cycloalkylalkylgruppe, eine Arylgruppe oder eine Arylalkylgruppe darstellt,
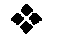 oder eine Gruppe 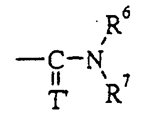 bedeutet, in der
T' ein Schwefelatom oder ein Sauerstoffatom darstellt,
R⁶ ein Wasserstoffatom oder eine geradkettige oder verzweigte (C₁-C₆)-Alkylgruppe bedeutet,
und R⁷ ein Wasserstoffatom, eine geradkettige oder verzweigte gegebenenfalls substituierte (C₁-C₆)-Alkylgruppe, eine geradkettige oder verzweigte gegebenenfalls substituierte (C₂-C₆)-Alkenylgruppe, eine geradkettige oder verzweigte gegebenenfalls substituierte (C₂-C₆)-Alkinylgruppe, eine (C₃-C₈)-Cycloalkylgruppe, eine substituierte (C₃-C₈)-Cycloalkylgruppe, eine Cycloalkylalkylgruppe, eine substituierte Cycloalkylalkylgruppe, eine Arylgruppe oder eine Arylalkylgruppe darstellt, bedeutet,
◆ R ein Wasserstoffatom, eine geradkettige oder verzweigte gegebenenfalls substituierte (C₁-C₆)-Alkylgruppe, eine Arylgruppe oder eine Arylalkylgruppe bedeutet,
◆ die Darstellung 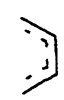 bedeutet, daß diese Bindungen einfach oder doppelt sein können mit der Maßgabe, daß zwei benachbarte Bindungen nicht gleichzeitig Doppelbindungen sein können und die Wertigkeit der Atome berücksichtigt ist,
mit der Maßgabe, daß:
- der Begriff "Aryl" für eine Phenyl- oder Naphthylgruppe steht, die gegebenenfalls durch ein oder mehrere Halogenatome, OH-Gruppen, geradkettige oder verzweigte (C₁-C₆)-Alkylgruppen, geradkettige oder verzweigte (C₁-C₆)-Alkoxygruppen, Cyanogruppen, Nitrogruppen, Aminogruppen oder Trihalogenalkylgruppen substituiert sein können,
- der Begriff "Arylalkyl" für eine geradkettige oder verzweigte (C₁-C₆)-Alkylgruppe, die durch eine Arylgruppe, wie sie oben definiert worden ist, substituiert ist, steht,
- der Begriff "Cycloalkylalkyl" für eine geradkettige oder verzweigte (C₁-C₆)-Alkylgruppe, die durch eine oder mehrere (C₃-C₈)-Cycloalkylgruppen substituiert ist, steht,
- der Begriff substituiert unter Bezug auf die Begriffe "Alkyl", "Alkenyl" und "Alkinyl" bedeutet, daß diese Gruppe durch ein oder mehrere Halogenatome, OH-Gruppen oder Alkoxygruppen substituiert ist,
- der Begriff substituiert unter Bezug auf die Begriffe "Cycloalkyl" und "Cycloalkylalkyl" bedeutet, daß der cyclische Teil durch ein oder mehrere Halogenatome, geradkettige oder verzweigte (C₁-C₆)-Alkylgruppen, geradkettige oder verzweigte (C₁-C₆)-Alkoxygruppen, Phenylgruppen, Hydroxygruppen oder Oxogruppen substituiert ist,
mit der Maßgabe; daß:
. wenn X und Z gleichzeitig eine Gruppe CH₂, R ein Wasserstoffatom und A eine Gruppe NR¹R² darstellen, Y kein Sauerstoffatom bedeutet,
. wenn Z ein Sauerstoffatom darstellt, n von Null verschieden ist,
. wenn Z ein Sauerstoffatom und n 1 bedeuten, A keine Gruppe CONEt₂ darstellt,
. wenn Z eine CH₂-Gruppe, n 1 und A eine Gruppe -NR¹CSNR⁴R⁵ darstellen, R⁵ keine Arylgruppe bedeutet,
wenn X und Y gleichzeitig ein Sauerstoffatom darstellen, die punktierten Bindungen gesättigt sind, R ein Sauerstoffatom oder eine Gruppe CH₂OH darstellt, A von einer Gruppe NR¹R^{2a} verschieden ist, in der R¹ die oben angegebenen Bedeutungen besitzt und R^{2a} eine gegebenenfalls substituierte Benzoylgruppe bedeutet,
deren Enantiomere und Diastereoisomere sowie deren Additionssalze mit einer pharmazeutisch annehmbaren Säure oder Base.

2. Verbindungen der Formel (I) nach Anspruch 1, worin R ein Wasserstoffatom bedeutet, deren Enantiomere und Diastereoisomere sowie deren Additionssalze mit einer pharmazeutisch annehmbaren Säure oder Base.

3. Verbindungen der Formel (I) nach Anspruch 1, worin X und Y gleichzeitig ein Sauerstoffatom bedeuten, deren Enantiomere und Diastereoisomere sowie deren Additionssalze mit einer pharmazeutisch annehmbaren Säure oder Base.

4. Verbindungen der Formel (I) nach Anspruch 1, worin X ein Schwefelatom und Y ein Sauerstoffatom bedeuten, deren Enantiomere und Diastereoisomere sowie deren Additionssalze mit einer pharmazeutisch annehmbaren Säure oder Base.

5. Verbindungen der Formel (I) nach Anspruch 1, worin X ein Schwefelatom oder ein Sauerstoffatom und Y eine Gruppe CHq (worin q 0, 1 oder 2 darstellt) bedeuten, deren Enantiomere und Diastereoisomere sowie deren Additionssalze mit einer pharmazeutisch annehmbaren Säure oder Base.

6. Verbindungen der Formel (I) nach Anspruch 1, worin Z ein Sauerstoffatom bedeutet, deren Enantiomere und Diastereoisomere sowie deren Additionssalze mit einer pharmazeutisch annehmbaren Säure oder Base.

7. Verbindungen der Formel (I) nach Anspruch 1, worin Z eine CH₂-Gruppe bedeutet, deren Enantiomere und Diastereoisomere sowie deren Additionssalze mit einer pharmazeutisch annehmbaren Säure oder Base.

8. Verbindungen der Formel (I) nach Anspruch 1, worin A eine Gruppe NR¹COR³ bedeutet, deren Enantiomere und Diastereoisomere sowie deren Additionssalze mit einer pharmazeutisch annehmbaren Säure oder Base.

9. Verbindungen der Formel (I) nach Anspruch 1, worin A eine Gruppe CONR⁶R⁷ bedeutet, deren Enantiomere und Diastereoisomere sowie deren Additionssalze mit einer pharmazeutisch annehmbaren Säure oder Base.

10. Verbindung der Formel (I) nach Anspruch 1, nämlich N-Methyl-[4-(2,3-dihydro-1,4-benzoxathiin-5-yl)]-butanamid, deren Enantiomere und Diastereoisomere sowie deren Additionssalze mit einer pharmazeutisch annehmbaren Säure oder Base.

11. Verbindung der Formel (I) nach Anspruch 1, nämlich N-Ethyl-[4-(2,3-dihydro-1,4-benzoxathiin-5-yl)]-butanamid, deren Enantiomere und Diastereoisomere sowie deren Additionssalze mit einer pharmazeutisch annehmbaren Säure oder Base.

12. Verbindung der Formel (I) nach Anspruch 1, nämlich N-[3-(1,4-Benzodioxin-5-yl)-propyl]-n-butanamid, deren Enantiomere und Diastereoisomere sowie deren Additionssalze mit einer pharmazeutisch annehmbaren Säure oder Base.

13. Verbindung der Formel (I) nach Anspruch 1, nämlich N-Methyl-[4-(2,3-dihydro-1,4-benzodioxin-5-yl)]-butanamid, deren Enantiomere und Diastereoisomere sowie deren Additionssalze mit einer pharmazeutisch annehmbaren Säure oder Base.

14. Verfahren zur Herstellung der Verbindungen der Formel (I) nach Anspruch 1, **dadurch gekennzeichnet, daß** man als Ausgangsprodukt eine Verbindung der Formel (II) verwendet: 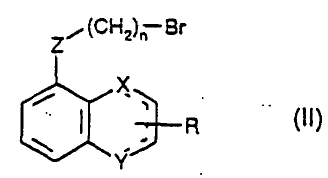 in der X, Y, Z, R und n die oben angegebenen Bedeutungen besitzen,
◆ welche man nacheinander der Einwirkung eines Phthalimids und dann einer Hydrazinolyse unterwirft zur Bildung der Verbindung der Formel (III): 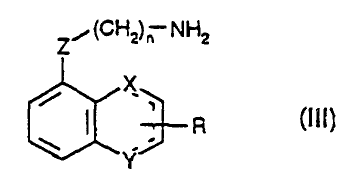 In der X, Y, Z, n und R die oben angegebenen Bedeutungen besitzen,
welche Verbindung (III) auch ausgehend von der Verbindung (II) durch Einwirkung eines Cyanid- oder Azidsalzes gefolgt von einer Reduktion erhalten werden kann,
welche man:
- mit einem Acylchlorid der Formel (IV): 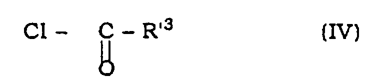 in der R'³ die oben angegebenen Bedeutungen besitzt, oder dem entsprechenden (gemischten oder symmetrischen) Säureanhydrid kondensiert,
zur Bildung der Verbindung der Formel (I/a), einem Sonderfall der Verbindungen der Formel (I): 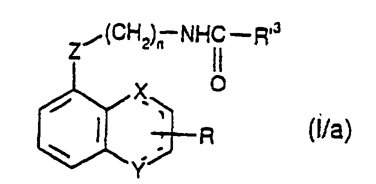 in der X, Y, Z, n, R und R'³ die oben angegebenen Bedeutungen besitzen, welche der Einwirkung eines Thionierungsmittels unterworfen werden kann zur Bildung der Verbindung der Formel (I/b), einem Sonderfall der Verbindungen der Formel (I): 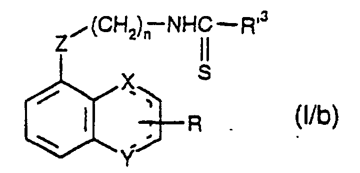 in der X, Y, Z, n, R und R'³ die oben angegebenen Bedeutungen besitzen,
- oder mit einer Verbindung der Formel (V) kondensiert
T = C = N - R⁵ (V)
in der T und R⁵ die oben angegebenen Bedeutungen besitzen,
zur Bildung der Verbindung der Formel (I/c), einem Sonderfall der Verbindungen der Formel (I): 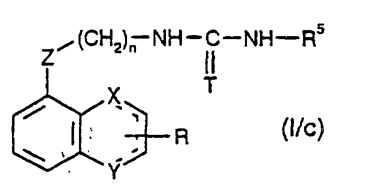 in der X, Y, Z, n, R, T und R⁵ die oben angegebenen Bedeutungen besitzen,
wobei die Gesamtheit der Verbindungen (I/a), (I/b) und (I/c) die Verbindungen der Formel (I/d) bilden, einem Sonderfall der Verbindungen der Formel (I): 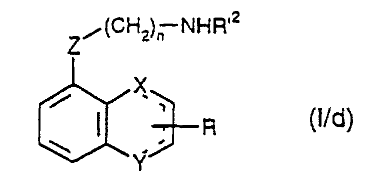 in der X, Y, Z, n und R die oben angegebenen Bedeutungen besitzen und R'² eine Gruppe 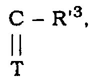 in der T und R'³ die oben angegebenen Bedeutungen besitzen,
oder eine Gruppe 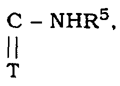 worin T und R⁵ die oben angegebenen Bedeutungen besitzen, darstellt,
welche Verbindung (I/d) man mit Hilfe einer klassischen Alkylierungstechnik mit einer Verbindung der Formel (VI):
Alk - W (VI)
in der Alk eine geradkettige oder verzweigte (C₁-C₆)-Alkylgruppe und W eine austretende Gruppe, wie ein Halogenatom oder eine Tosylgruppe bedeuten.
oder mit einem Dialkylsulfat alkylieren kann
zur Bildung der Verbindung der Formel (I/e), einem Sonderfall der Verbindungen der Formel (I): 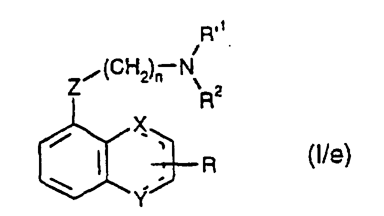 in der X, Y, Z, n, R und R² die oben angegebenen Bedeutungen besitzen und R'¹ eine geradkettige oder verzweigte (C₁-C₆)-Alkylgruppe bedeutet,
◆ oder welche man nacheinander der Einwirkung eines Carbonsäuresalzes und dann einer Verseifung unterwirft zur Bildung der Verbindung der Formel (VII): 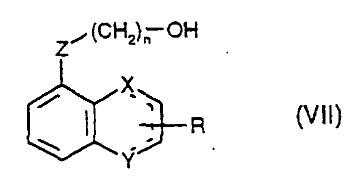 in der X, Y, Z, n und R die oben angegebenen Bedeutungen besitzen, welche:
- entweder zu dem entsprechenden Aldehyd oxidiert wird zur Bildung der Verbindung der Formel (VIII): 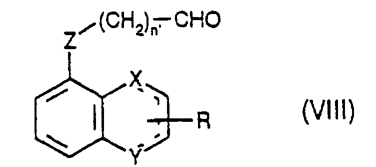 in der X, Y, Z und R die oben angegebenen Bedeutungen besitzen und n' 0, 1, 2 oder 3 bedeutet,
welche zu der entsprechenden Säure oxidiert und dann der Einwirkung eines Amins H₂NR⁷, in der R⁷ die oben angegebenen Bedeutungen besitzt, unterworfen wird,
zur Bildung der Verbindung der Formel (I/f), einem Sonderfall der Verbindungen der Formel (I): 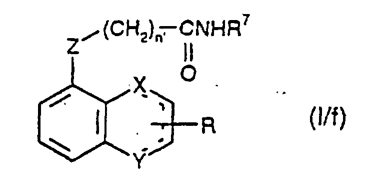 in der X, Y, Z, n', R und R⁷ die oben angegebenen Bedeutungen besitzen, welche der Einwirkung eines Thionierungsmittels unterworfen werden kann zur Bildung der Verbindung (I/g), einem Sonderfall der Verbindungen der Formel (I); 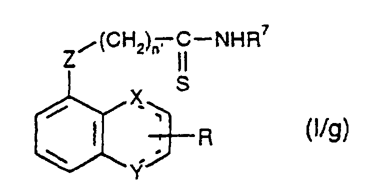 in der X, Y, Z, n', R und R⁷ die oben angegebenen Bedeutungen besitzen, wobei die Gesamtheit der Verbindungen (I/f) und (I/g) die Verbindungen der Formel (I/h) bilden: 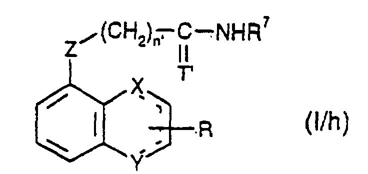 in der X, Y, Z, T', n', R und R⁷ die oben angegebenen Bedeutungen besitzen, welche Verbindung (I/h) mit Hilfe einer klassischen Alkylierungstechnik alkyliert werden kann zur Bildung der Verbindung der Formel (I/i), einem Sonderfall der Verbindungen der Formel (I): 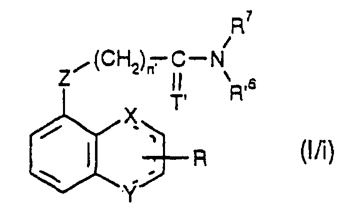 in der X, Y, Z, T', n', R und R⁷ die oben angegebenen Bedeutungen besitzen und R'⁶ eine geradkettige oder verzweigte (C₁-C₆)-Alkylgruppe darstellt,
- oder in eine austretende Gruppe, wie ein Tosylat, umgewandelt werden und dann
. mit einem Cyanid substituiert werden kann zur Bildung der Verbindung der Formel (IX): 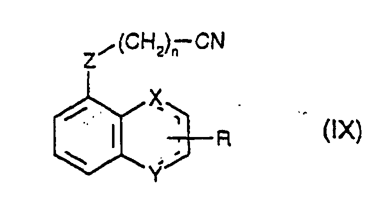 in der X, Y, Z, R und n die oben angegebenen Bedeutungen besitzen, welche nacheinander einer Hydrolyse und dann einer Kondensation mit einem Amin H₂NR⁷ unterworfen werden zur Bildung der Verbindung der Formel (I/j), einem Sonderfall der Verbindungen der Formel (I): 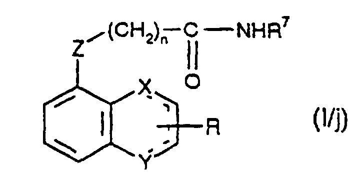 in der X, Y, Z, n, R und R⁷ die oben angegebenen Bedeutungen besitzen, welche der Einwirkung eines Thionierungsmittels unterworfen werden kann zur Bildung der Verbindung (I/k), einem Sonderfall der Verbindungen der Formel (I): 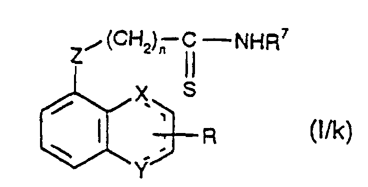 in der X, Y, Z, n, R und R⁷ die oben angegebenen Bedeutungen besitzen, wobei die Verbindungen (I/j) und (I/k) die Verbindung der Formel (I/l) bilden, einem Sonderfall der Verbindungen der Formel (I): 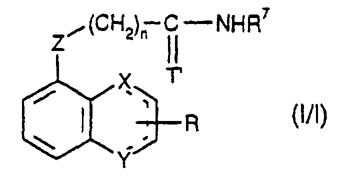 in der X, Y, Z, n, T', R und R⁷ die oben angegebenen Bedeutungen besitzen, welche mit Hilfe einer klassischen Alkylierungstechnik alkyliert werden kann zur Bildung der Verbindung der Formel (I/m), einem Sonderfall der Verbindungen der Formel (I): 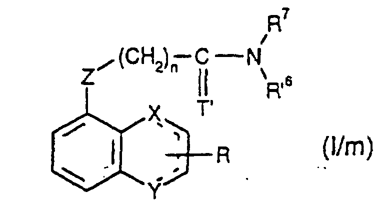 in der X, Y, Z, n, T', R und R⁷ die oben angegebenen Bedeutungen besitzen und R'⁶ eine geradkettige oder verzweigte (C₁-C₆)-Alkylgruppe darstellt,
. oder mit einem Azid substituiert werden kann gefolgt von einer Reduktion zur Bildung der Verbindung der Formel (III),
wobei die Gesamtheit der Verbindungen (I/a) bis (I/m), welche die Verbindungen der allgemeinen Formel (I) bilden, mit Hilfe einer klassischen Reinigungsmethode gereinigt werden können, gegebenenfalls mit Hilfe einer klassischen Trennmethode in ihre Isomeren aufgetrennt werden können und gewünschtenfalls in ihre Additionssalze mit einer pharmazeutisch annehmbaren Säure oder Base umgewandelt werden können.

15. Pharmazeutische Zubereitungen enthaltend die Produkte der Formel (I) nach einem der Ansprüche 1 bis 13 oder eines ihrer Additionssalze mit einer pharmazeutisch annehbmaren Säure oder Base in Kombination mit einem oder mehreren pharmazeutisch annehmbaren Trägermaterialien.

16. Pharmazeutische Zubereitungen nach Anspruch 15 zur Behandlung von Störungen, die mit dem melatoninergischen System verbunden sind.

## Claims

1. Compounds of formula (I) : 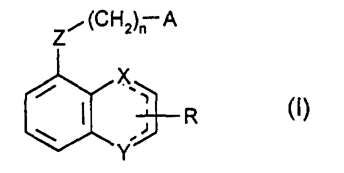 wherein:
◆ X and Y, which may be identical or different, each represents a sulphur atom, an oxygen atom, a CH_{q} group (wherein q is 0, 1 or 2) or an SO or SO₂ group, with the proviso that X and Y cannot simultaneously represent a CH_{q} group (wherein q is 0, 1 or 2),
◆ Z represents an oxygen atom or a CH₂ group,
◆ n is 0, 1, 2, 3 or 4,
◆ A represents
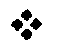 a grouping 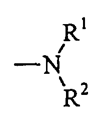 wherein
R¹ represents a hydrogen atom or a linear or branched (C₁-C₆)-alkyl group,
and R² represents a grouping 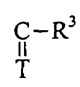 wherein T represents
a sulphur atom or an oxygen atom and R³ represents
- an R'³ group representing a hydrogen atom, an optionally substituted linear or branched (C₁-C₆)-alkyl group, an optionally substituted linear or branched (C₂-C₆)-alkenyl group, an optionally substituted linear or branched (C₂-C₆)-alkynyl group, a (C₃-C₈)-cycloalkyl group, a substituted (C₃-C₈)-cycloalkyl group, a cycloalkylalkyl group, a substituted cycloalkylalkyl group, an aryl group or an arylalkyl group,
- or a grouping 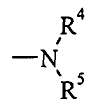
wherein R⁴ represents a hydrogen atom
or a linear or branched (C₁-C₆)-alkyl group and R⁵ represents a hydrogen atom, an R'⁵ group representing an optionally substituted linear or branched (C₁-C₆)-alkyl group, an optionally substituted linear or branched (C₂-C₆)-alkenyl group, an optionally substituted linear or branched (C₂-C₆)-alkynyl group, a (C₃-C₈)-cycloalkyl group, a substituted (C₃-C₈)-cycloalkyl group, a cycloalkylalkyl group, a substituted cycloalkylalkyl group, an aryl group or an arylalkyl group,
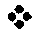 or a grouping 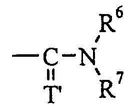 wherein
T' represents a sulphur atom or an oxygen atom,
R⁶ represent a hydrogen atom or a linear or branched (C₁-C₆)-alkyl group
and R⁷ represents a hydrogen atom, an optionally substituted linear or branched (C₁-C₆)-alkyl group, an optionally substituted linear or branched (C₂-C₆)-alkenyl group, an optionally substituted linear or branched (C₂-C₆)-alkynyl group, a (C₃-C₈)-cycloalkyl group, a substituted (C₃-C₈)-cycloalkyl group, a cycloalkylalkyl group, a substituted cycloalkylalkyl group, an aryl group or an arylalkyl group,
◆ R represents a hydrogen atom, an optionally substituted linear or branched (C₁-C₆)-alkyl group, an aryl group or an arylalkyl group,
◆ the representation 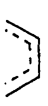 denotes that those bonds may be single or double, it being understood that two adjacent bonds cannot simultaneously be double and that the valency of the atoms is respected,
and wherein :
- the term "aryl" denotes a phenyl or naphthyl group optionally substituted by one or more halogen atoms, OH, linear or branched (C₁-C₆)-alkyl, linear or branched (C₁-C₆)-alkoxy, cyano, nitro, amino or trihaloalkyl groups,
- the term "arylalkyl" denotes a linear or branched (C₁-C₆)-alkyl group substituted by an aryl group as defined above,
- the term "cycloalkylalkyl" denotes a linear or branched (C₁-C₆)-alkyl group substituted by one or more (C₃-C₈)-cycloalkyl groups,
- the term "substituted" governing the terms "alkyl", "alkenyl" and "alkynyl" denotes that that group is substituted by one or more halogen atoms, OH groups or alkoxy groups,
- the term "substituted" governing the terms "cycloalkyl" and "cycloalkylalkyl" denotes that the cyclic moiety is substituted by one or more halogen atoms, linear or branched (C₁-C₆)-alkyl, linear or branched (C₁-C₆)-alkoxy, phenyl, hydroxy or oxo groups,
with the proviso that
. when X and Z simultaneously represent a CH₂ group, R represents a hydrogen atom and A represents a NR¹R² group, Y cannot represent an oxygen atom,
. when Z represents an oxygen atom, n is other than zero,
. when Z represents an oxygen atom and n is 1, A cannot represent a CONEt₂ grouping,
. when Z represents a CH₂ group, n is 1 and A represents a -NR¹CSNR⁴R⁵ grouping, R⁵ cannot represent an aryl group,
. when X and Y simultaneously represent an oxygen atom and the broken-line bonds are saturated, and R represents a hydrogen atom or a CH₂OH group, then A is other than an NR¹R^{2a} grouping wherein R' is as defined hereinbefore and R^{2a} represents an optionally substituted benzoyl group,
their enantiomers and diastereoisomers, and also the addition salts thereof with a pharmaceutically acceptable acid or base.

2. Compounds of formula (I) according to claim 1, wherein R represents a hydrogen atom, their enantiomers and diastereoisomers, and also the addition salts thereof with a pharmaceutically acceptable acid or base.

3. Compounds of formula (I) according to claim 1, wherein X and Y simultaneously represent an oxygen atom, their enantiomers and diastereoisomers, and also the addition salts thereof with a pharmaceutically acceptable acid or base.

4. Compounds of formula (I) according to claim 1, wherein X represents a sulphur atom and Y represents an oxygen atom, their enantiomers and diastereoisomers, and also the addition salts thereof with a pharmaceutically acceptable acid or base.

5. Compounds of formula (I) according to claim 1, wherein X represents a sulphur or oxygen atom and Y represents a CH_{q} group (wherein q is 0, 1 or 2), their enantiomers and diastereoisomers, and also the addition salts thereof with a pharmaceutically acceptable acid or base.

6. Compounds of formula (I) according to claim 1, wherein Z represents an oxygen atom, their enantiomers and diastereoisomers, and also the addition salts thereof with a pharmaceutically acceptable acid or base.

7. Compounds of formula (I) according to claim 1, wherein Z represents a CH₂ group, their enantiomers and diastereoisomers, and also the addition salts thereof with a pharmaceutically acceptable acid or base.

8. Compounds of formula (I) according to claim 1, wherein A represents a grouping NR¹COR³, their enantiomers and diastereoisomers, and also the addition salts thereof with a pharmaceutically acceptable acid or base.

9. Compounds of formula (I) according to claim 1, wherein A represents a grouping CONR⁶R⁷, their enantiomers and diastereoisomers, and also the addition salts thereof with a pharmaceutically acceptable acid or base.

10. Compound of formula (I) according to claim 1 which is N-methyl-[4-(2,3-dihydro-1,4-benzoxathiin-5-yl)]butanamide, its enantiomers and diastereoisomers, and also the addition salts thereof with a pharmaceutically acceptable acid or base.

11. Compound of formula (I) according to claim 1 which is N-ethyl-[4-(2,3-dihydro-1,4-benzoxathiin-5-yl)]butanamide, its enantiomers and diastereoisomers, and also the addition salts thereof with a pharmaceutically acceptable acid or base.

12. Compound of formula (I) according to claim 1 which is N-[3-(1,4-benzodioxin-5-yl)-propyl]-n-butanamide, its enantiomers and diastereoisomers, and also the addition salts thereof with a pharmaceutically acceptable acid or base.

13. Compound of formula (I) according to claim 1 which is N-methyl-[4-(2,3-dihydro-1,4-benzodioxin-5-yl)]butanamide, its enantiomers and diastereoisomers, and also the addition salts thereof with a pharmaceutically acceptable acid or base.

14. Process for the preparation of compounds of formula (I) according to claim 1,
**characterised in that** there is used as starting material a compound of formula (II): 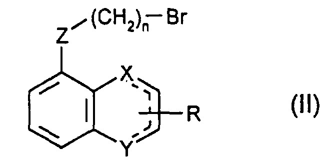 wherein X, Y, Z, R and n are as defined hereinbefore,
◆ which is subjected in succession to the action of a phthalimide and to hydrazinolysis to yield a compound of formula (III): 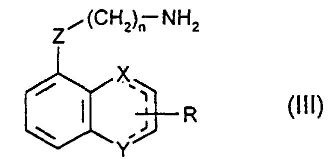 wherein X, Y, Z, n and R are as defined hereinbefore,
it being possible for compound (III) furthermore to be obtained starting from compound (II) by the action of a cyanide salt or an azide followed by reduction,
which compound (III) is condensed with:
- an acyl chloride of formula (IV) 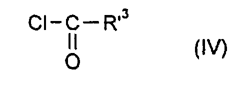 wherein R'³ is as defined hereinbefore, or with a corresponding acid anhydride (mixed or symmetrical),
to obtain a compound of formula (I/a): 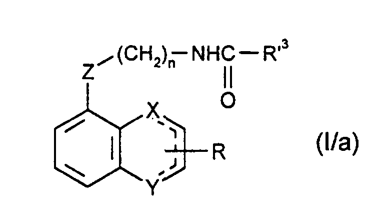 wherein X, Y, Z, n, R and R'³ are as defined hereinbefore, a particular case of the compounds of formula (I),
which may be subjected to the action of a thionisation agent to obtain a compound of formula (I/b): 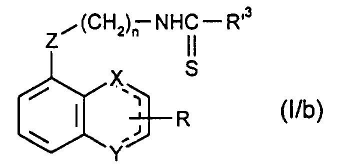 wherein X, Y, Z, n, R and R'³ are as defined hereinbefore, a particular case of the compounds of formula (I),
- or with a compound of formula (V)
T=C=N-R⁵ (V)
wherein T and R⁵ are as defined hereinbefore,
in order to obtain a compound of formula (I/c): 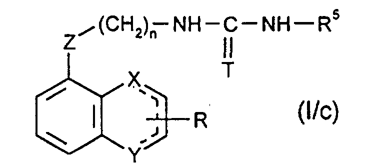 wherein X, Y, Z, n, R, T et R⁵ are as defined hereinbefore, a particular case of the compounds of formula (I),
the totality of the compounds (I/a), (I/b) and (I/c) constituting the compound of formula (I/d): 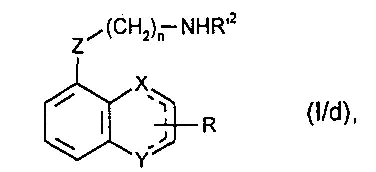 a particular case of the compounds of formula I, wherein X, Y, Z, n and Rare as defined hereinbefore and R'² represents
a grouping 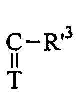 wherein T and R'³ are as defined hereinbefore
or a grouping 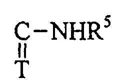 wherein T and R⁵ are as defined hereinbefore, which compound (I/d) may be alkylated according to a conventional alkylation technique with a compound of formula (VI):
Alk-W (VI)
wherein Alk represents a linear or branched (C₁-C₆)-alkyl group and W represents a leaving group, such as a halogen atom or a tosyl group,
or with a dialkyl sulphate,
to yield a compound of formula (I/e): 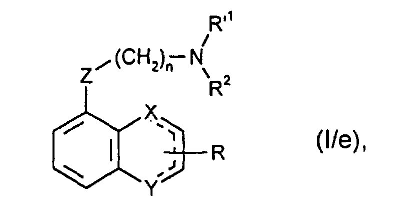 a particular case of the compounds of formula (I), wherein X, Y, Z, n, R and R² are as defined hereinbefore and R'¹ represents a linear or branched (C₁-C₆)-alkyl group,
◆ or which is subjected in succession to the action of a carboxylic acid salt then to hydrolysis to yield a compound of formula (VII): 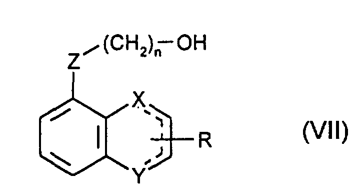 wherein X, Y, Z, n and R are as defined hereinbefore,
which is :
- either oxidised to the corresponding aldehyde to obtain a compound of formula (VIII): 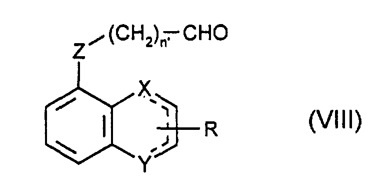 wherein X, Y, Z and R are as defined hereinbefore, and n' is 0, 1, 2 or 3,
which is oxidised to the corresponding acid and then subjected to the action of an amine H₂NR⁷ wherein R⁷ is as defined hereinbefore,
to yield a compound of formula (I/f): 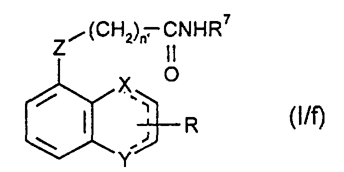 wherein X, Y, Z, n', R and R⁷ are as defined hereinbefore, a particular case of the compounds of formula (I),
which may be subjected to the action of a thionisation agent to obtain a compound (I/g): 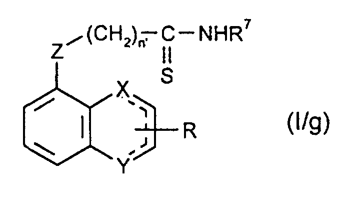 wherein X, Y, Z, n', R and R⁷ are as defined hereinbefore, a particular case of the compounds of formula (I),
the totality of the compounds (I/f) and (I/g) constituting the compound of formula (I/h): 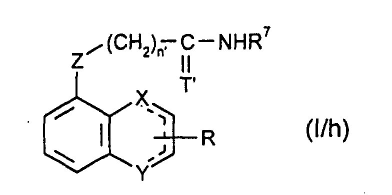 wherein X, Y, Z, T', n', R and R⁷ are as defined hereinbefore,
which compound (I/h) may be alkylated according to a conventional alkylation technique to yield a compound of formula (I/i): 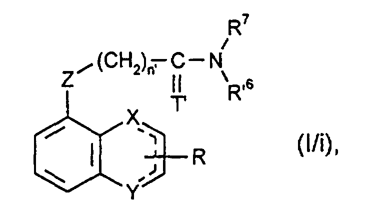 a particular case of the compounds of formula (I), wherein X, Y, Z, T', n', R and R⁷ are as defined hereinbefore and R'⁶ represents a linear or branched (C₁-C₆)-alkyl group,
- or converted into a leaving group, such as a tosylate, then substituted
. with a cyanide to yield a compound of formula (IX) : 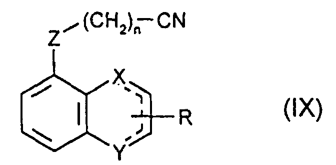 wherein X, Y, Z, R and n are as defined hereinbefore,
which is subjected in succession to hydrolysis and to condensation with an amine H₂NR⁷ to yield a compound of formula (I/j): 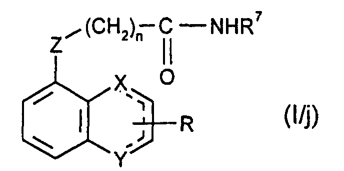 wherein X, Y, Z, n, R and R⁷ are as defined hereinbefore, a particular case of the compounds of formula (I),
which may be subjected to the action of a thionisation agent to obtain a compound (I/k): 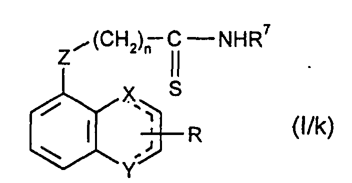 wherein X, Y, Z, n, R and R⁷ are as defined hereinbefore, a particular case of the compounds of formula (I),
the totality of the compounds (I/j) and (I/k) constituting the compound of formula (I/l): 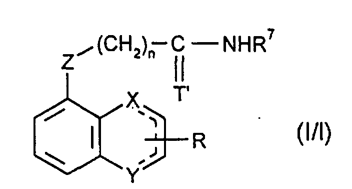 wherein X, Y, Z, n, T', R and R⁷ are as defined hereinbefore, a particular case of the compounds of formula (I)
which may be alkylated according to a conventional alkylation technique to yield a compound of formula (I/m): 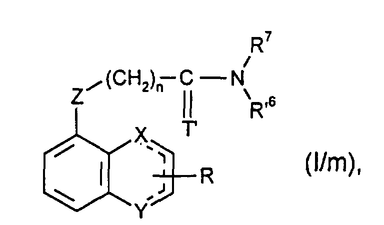 a particular case of the compounds of formula (I), wherein X, Y, Z, n, T', R and R⁷ are as defined hereinbefore and R'⁶ represents a linear or branched (C₁-C₆)-alkyl group,
. or with an azide followed by reduction, to yield a compound of formula (III),
the totality of the compounds (I/a) to (I/m) constituting the compounds of the general formula (I), which may be purified according to a conventional purification technique, are separated, optionally, into their isomers according to a conventional separation technique, or are converted, if desired, into the addition salts thereof with a pharmaceutically acceptable acid or base.

15. Pharmaceutical compositions comprising the products of formula (I) according to any one of claims 1 to 13 or an addition salt thereof with a pharmaceutically acceptable acid or base in combination with one or more pharmaceutically acceptable excipients:

16. Pharmaceutical compositions according to claim 15 for use in the treatment of disorders associated with the melatoninergic system.
